# EUROPEAN PATENT APPLICATION

(11) **EP 4 660 196 A1**
(43) Date of publication of application: **10.12.2025**
(21) Application number: 24749682.1
(22) Date of filing: 30.01.2024
(51) Int. Cl.: C07D 487/04, A61K 31/519, A61P 35/00, A61P 29/00, A61P 19/02, A61P 17/00, A61P 1/04, A61P 1/00, A61P 35/02

(54) **CRYSTAL FORM OF COMPOUND, PREPARATION METHOD THEREFOR AND USE THEREOF**

(30) Priority: 31.01.2023 CN 202310048113
(71) Applicant: Felicamed Biotechnology Co., Ltd., Yangjiang, Guangdong 529500 (CN)
(72) Inventor: AN, Na, Zhuhai, Guangdong 519031 (CN); LI, Danni, Zhuhai, Guangdong 519031 (CN); SONG, Min, Zhuhai, Guangdong 519031 (CN); LIN, Xingyu, Zhuhai, Guangdong 519031 (CN); LU, Tingting, Zhuhai, Guangdong 519031 (CN); ZHANG, Xue, Zhuhai, Guangdong 519031 (CN)
(74) Representative: Michalski Hüttermann & Partner Patentanwälte mbB
(86) International application number: PCT/CN2024/074718
(87) International publication number: WO 2024/160203

(57) **Abstract**

Disclosed is a crystal form of a compound, and a preparation and use of the compound. The compound has a structure represented by formula I. The crystal form has high solid-state stability and fluidity, low hygroscopicity, and high solubility, which are conducive to preparation and storage of a pharmaceutical preparation. Furthermore, the crystal form has very good pharmacokinetic properties and high safety during continuous administration. It may be administered through a variety of routes (such as injection and oral administration), offers a variety of preparation forms, and particularly high oral bioavailability, making it highly valuable for practical applications. The crystal form may be taken as a Janus kinase (JAK) inhibitor, and used in development of drugs for preventing and/or treating inflammatory diseases, tumors, autoimmune diseases, and allergic diseases in humans and/or animals.

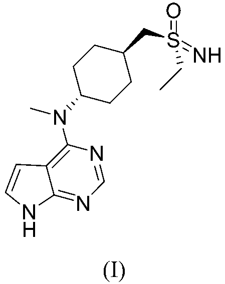

## Description

### FIELD

The present application relates to the field of pharmaceutical chemistry polymorph research, and specifically, relates to a crystal form of a compound represented by formula I, and a preparation method and use thereof.

### BACKGROUND

The Janus kinase-signal transducer and activator of transcription (JAK-STAT) signaling pathway is a signal transduction pathway stimulated by cytokines and is involved in many important biological processes such as cell proliferation, differentiation, apoptosis, and immune regulation. Compared to other signaling pathways, this signaling pathway has a relatively simple transmission process. It is mainly composed of three components, namely, a tyrosine kinase-related receptor, tyrosine kinase JAK, and transcription factor STAT.

A JAK inhibitor may selectively inhibit JAK and block the JAK-STAT pathway. Currently, JAK inhibitor drugs approved by the U.S. Food and Drug Administration (FDA) include tofacitinib, ruxolitinib, oclacitinib, and baricitinib. Although oclacitinib has a good therapeutic effect on allergic skin diseases in pet dogs, it has little effect on cytokines that are not involved in the activation of JAK1. Its effect on allergic reactions is limited to the stage of inhibiting the release of allergic mediators. It cannot directly block the binding of allergic mediators to related receptors, and thus cannot fundamentally block the occurrence and development of allergic skin diseases. Therefore, the scope of application of oclacitinib is limited. Baricitinib is a selective JAK1 and JAK2 inhibitor with IC50 values of 5.9 nM and 5.7 nM, respectively. It is approximately 70 and 10 times more selective than JAK3 and Tyk2, but has no inhibitory effect on c-Met and Chk2, and its indications are relatively single.

The Chinese Patent Application No. CN111499641B discloses a JAK inhibitor and a preparation method thereof. The JAK inhibitor has a high JAK inhibitory activity, and is expected to be used to prevent and/or treat inflammatory diseases and cancer in humans and/or animals. While therapeutic activity is the primary property of therapeutic agents, the solid-state form (namely, crystalline or amorphous form) of a candidate drug is also critical to its pharmacological properties and its development as a viable active pharmaceutical ingredient (API).

### SUMMARY

To address the limitations of the relative technology, the present application discloses a crystal form of a compound represented by formula I, and a preparation method and use thereof.

The present application provides a crystal form of a compound represented by formula I, 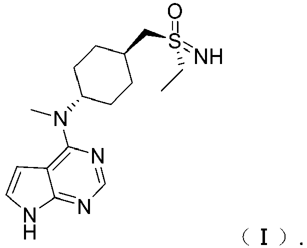 an X-ray powder diffraction (XRPD) pattern of the crystal form I (using Cu-Kα radiation) exhibits characteristic peaks at positions corresponding to at least three (such as three, four, five, six, seven, or particularly all) 20 values selected from approximately 12.4°±0.2°, 14.6°±0.2°, 16.7°±0.2°, 17.2°±0.2°, 20.3°±0.2°, 24.8°±0.2°, and 25.0°±0.2°.

Further, the XRPD pattern of the crystal form I further exhibits characteristic peaks at positions corresponding to at least three (such as three, four, five, six, seven, or particularly all) 2θ values selected from approximately 13.2°±0.2°, 15.2°±0.2°, 19.3°±0.2°, 21.2°±0.2°, 21.6°±0.2°, 24.0°±0.2°, and 27.0°±0.2°.

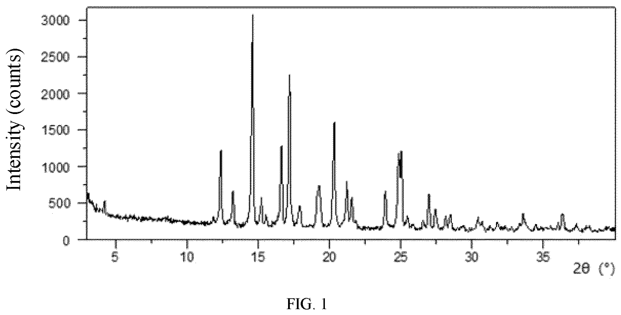

Further, the crystal form I has an XRPD pattern substantially same as that shown in FIG. 1.

Further, the DSC pattern of the crystal form I exhibits endothermic peaks at positions corresponding to 195°C to 210°C (such as approximately 195°C, 200°C, 205°C, or 210°C).

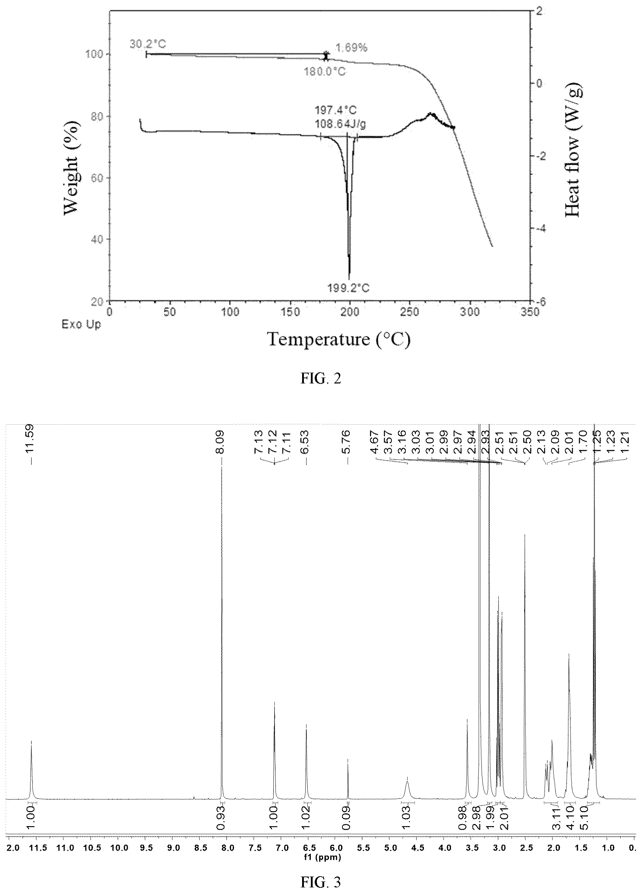

Further, the crystal form I has a differential scanning calorimetry (DSC) pattern that is substantially the same as that shown in FIG. 2.

Further, the TGA pattern of the crystal form I exhibits 1.7% (such as 1.0%, 1.5%, 1.7%, 2.0%, 2.5%, or 3.0%) weight loss during heating from 30°C to 180°C.

Further, the crystal form I has a thermal gravimetric analysis (TGA) pattern that is substantially the same as that shown in FIG. 2.

Further, the crystal form I is an anhydrous crystal form.

Further, when observed under a polarizing microscope, the crystal form I is a needle-shaped and/or long sheet-like crystals.

The present application further provides a preparation method of the crystal form I, including: taking an amorphous compound represented by formula I as a starting material to prepare the crystal form I. The crystal form I may be prepared by one or a combination selected from an antisolvent addition method, a gas-solid diffusion method, a suspension stirring method, a slow volatilization method, a slow cooling method, a gas-liquid permeation method, and anti-antisolvent addition method.

Further, the antisolvent addition method specifically includes the following steps: dissolving an amorphous raw material of the compound represented by formula I in a good solvent, followed by dropwise addition of an antisolvent under stirring until solid precipitation occurs.

Further, the good solvent is selected from one of methanol, 1,4-dioxane, trichloromethane, and dimethyl sulfoxide.

Further, the antisolvent is selected from one of methyl isobutyl ketone, isopropyl acetate, methyl tert-butyl ether, n-heptane, ethyl acetate, 2-butanone, meta-xylene, cyclopentyl methyl ether, toluene, anisole, and water.

In some embodiments of the present application, in the antisolvent addition method, the good solvent is methanol, and the antisolvent is selected from methyl isobutyl ketone, isopropyl acetate, and methyl tert-butyl ether.

In some other embodiments of the present application, in the antisolvent addition method, the good solvent is 1,4-dioxane, and the antisolvent is selected from n-heptane and ethyl acetate.

In some other embodiments of the present application, in the antisolvent addition method, the good solvent is trichloromethane, and the antisolvent is selected from n-heptane, meta-xylene, and cyclopentyl methyl ether.

In some other embodiments of the present application, in the antisolvent addition method, the good solvent is dimethyl sulfoxide, and the antisolvent is selected from toluene, anisole, and water.

Further, the gas-solid diffusion method specifically includes the following steps: mixing an amorphous raw material of the compound represented by formula I with a solvent, sealing, and placing at room temperature until a solid is precipitated.

Further, the suspension stirring method is selected from a room temperature suspension stirring method, a 50°C suspension stirring method, and a temperature cycling suspension stirring method.

Further, the room temperature suspension stirring method specifically includes the following steps: suspending an amorphous raw material of the compound represented by formula I in a solvent, and stirring at room temperature until a solid is precipitated.

Further, the 50°C suspension stirring method specifically includes the following steps: suspending an amorphous raw material of the compound represented by formula I in a solvent, performing suspension stirring at a temperature cycle of 50°C to 5°C for 2 to 4 days, and performing suspension stirring at 50°C again until a solid is precipitated.

Further, the temperature cycling suspension stirring method specifically includes the following steps: suspending an amorphous raw material of the compound represented by formula I in a solvent, stirring at 40°C to 60°C, cooling from the range of 40°C to 60°C to a range of 0°C to 10°C within 400 min to 500 min, stirring at 0°C to 10°C for 1 h to 3 h, heating to the range of 40°C to 60°C within 20 min to 40 min, stirring at 40°C to 60°C for 1 h to 3 h, repeating the foregoing steps (for example, twice), cooling to the range of 0°C to 10°C within 400 min to 500 min, and stirring at 0°C to 10°C until a solid is precipitated.

Further, the slow volatilization method specifically includes the following steps: dissolving an amorphous raw material of the compound represented by formula I in a solvent, shaking, filtering to obtain a filtrate, and slowly volatilizing until a solid is precipitated.

Further, the slow cooling method specifically includes the following steps: dissolving an amorphous raw material of the compound represented by formula I in a solvent, stirring at 40°C to 60°C, filtering after the solution is clarified, slowly cooling the filtrate from the range of 40°C to 60°C to a range of 0°C to 10°C, and collecting a precipitated solid.

Further, the gas-liquid permeation method specifically includes the following steps: placing an amorphous raw material of the compound represented by formula I into an open container, dissolving in a solvent, placing the open contain in a sealed container containing an antisolvent, and placing at room temperature until a solid is precipitated.

Further, the anti-antisolvent addition method specifically includes the following steps: dissolving an amorphous raw material of the compound represented by formula I in a good solvent, and adding an obtained solution to an antisolvent until a solid is precipitated.

The present application further provides a hydrochloride crystal form I of a compound represented by formula I.

Further, an XRPD pattern (using Cu-Kα radiation) of the hydrochloride crystal form I exhibits characteristic peaks at positions corresponding to at least three (such as three, four, five, six, seven, eight, nine and particularly all) 2θ values selected from approximately 6.2°±0.2°, 10.9°±0.2°, 12.3°±0.2°, 16.3°±0.2°, 17.2°±0.2°, 18.9°±0.2°, 19.4°±0.2°, 24.7°±0.2°, and 27.5°±0.2°.

Further, the XRPD pattern (using Cu-Kα radiation) of the hydrochloride crystal form I further exhibits characteristic peaks at positions corresponding to at least three (such as three, four, five, six, seven, eight, nine and particularly all) 20 values selected from approximately 11.6°±0.2°, 15.0°±0.2°, 18.4°±0.2°, 21.3°±0.2°, 23.3°±0.2°, 24.3°±0.2°, 25.6°±0.2° , 26.8°±0.2°, and 30.0°±0.2°.

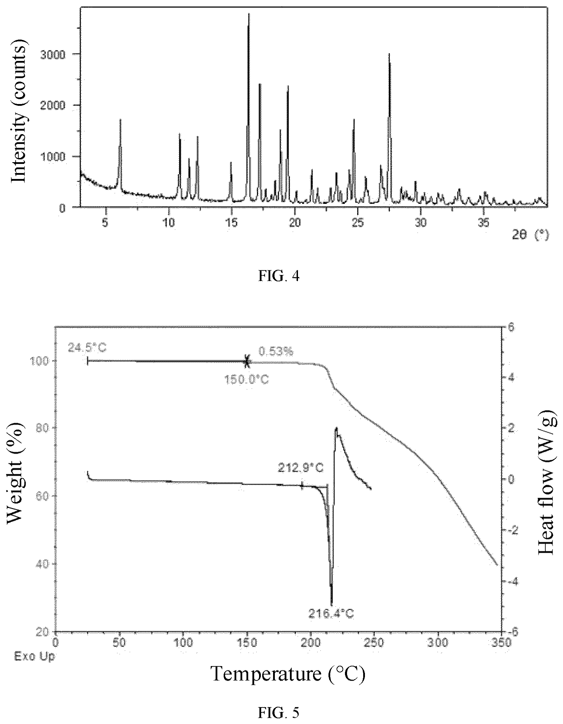

Further, the hydrochloride crystal form I has an XRPD pattern substantially same as that shown in FIG. 4.

Further, the DSC pattern of the hydrochloride crystal form I exhibits endothermic peaks at positions corresponding to 210°C to 220°C (such as approximately 210°C, 213°C, or 216°C).

Further, the hydrochloride crystal form I has a DSC pattern that is substantially the same as that shown in FIG. 5.

Further, the TGA pattern of the hydrochloride crystal form I exhibits 0.5% (such as 0.5%, 1.0%, 1.5%, 2.0%, 2.5%, or 3.0%) weight loss during heating from 25°C to 150°C.

Further, the hydrochloride crystal form I has a TGA pattern that is substantially the same as that shown in FIG. 5.

Further, when observed under a polarizing microscope, the hydrochloride crystal form I is a rod-shaped crystal.

The present application further provides a preparation method of the hydrochloride crystal form I, including: mixing the crystal form I (as described in the present application) with hydrochloric acid, and adding a solvent (stirring and centrifuging) to obtain the hydrochloride crystal form I.

Further, a molar ratio of the crystal form I to hydrochloric acid is 1: 1.

Further, the solvent is selected from one of ethanol, acetone/water (for example 19:1, v/v), ethyl acetate, and 2-methyltetrahydrofuran.

The present application further provides a pharmaceutical composition, including the crystal form I or the hydrochloride crystal form I of a compound represented by form I, and one or more pharmaceutically acceptable excipients.

Further, the excipient is selected from one or more of a carrier, a diluent, a binder, a lubricant, and a wetting agent. Preferably, the pharmaceutical composition contains a therapeutically effective amount of crystal form I or hydrochloride crystal form I of the compound represented by formula I.

Preferably, the pharmaceutical composition may be administered to a human and/or an animal.

Further, the pharmaceutical composition is suitable for gastrointestinal administration or non-gastrointestinal administration, such as intravenous administration, intramuscular administration, intradermal administration, and subcutaneous administration. Therefore, preferably, the pharmaceutical composition further includes an antioxidant, a buffer, a bacteriostat, a solute that renders the preparation isotonic with the blood of a recipient, and aqueous and non-aqueous sterile suspensions that may contain a suspending agent, a solubilizer, a thickener, a stabilizer, and a preservative.

Further, the pharmaceutical composition may be prepared into pharmaceutic preparation in the following form: syrup, an elixir, a suspension, powder, a granule, a tablet, a capsule, a lozenge, an aqueous solution, a cream, an ointment, a lotion, a gel, an emulsion, and the like.

Further, the pharmaceutic preparation is preferably in a unit dosage form. In this form, the pharmaceutic preparation is subdivided into unit doses containing an appropriate amount of active ingredient. The unit dosage form may be capsules, tablets, or any dosage forms. In addition, the unit dosage form may be a packaged preparation, such as a tablet, a capsule, or powder packaged in a vials or ampoule.

The amount of active ingredient in the unit dosage form may be changed or adjusted from 0.1 mg to 1000 mg, according to specific use and efficacy of the active component. If desired, the composition may also contain another suitable therapeutic agent.

The present application further provides use of the crystal form I or hydrochloride crystal form I of the compound represented by formula I in preparation of a JAK inhibitor or a drug for treating a disease associated with the Janus kinase signal transducer and activator of transcription (JAK-STAT) pathway.

The present application further provides use of the crystal form I or hydrochloride crystal form I of the compound represented by formula I in inhibition of JAK or treatment of a disease associated with the JAK-STAT pathway.

Further, the disease is selected from an inflammatory disease, a tumor, an autoimmune disease, and an allergic disease.

The present application further provides use of the crystal form I or the hydrochloride crystal form I of a compound represented by formula I in preparation of drugs for preventing and/or treating an inflammatory disease, a tumor, an autoimmune disease, and an allergic disease in humans and/or animals.

The present application further provides use of the crystal form I or the hydrochloride crystal form I of a compound represented by formula I for preventing and/or treating an inflammatory disease, a tumor, an autoimmune disease, and an allergic disease in humans and/or animals.

Further, the inflammatory disease is selected from rheumatoid arthritis, canine dermatitis, psoriasis, ulcerative colitis, and Crohn's disease.

Further, the tumor is malignant tumor (cancer); furthermore, the tumor is selected from myelofibrosis, polycythemia vera, essential thrombocythemia, chronic myelogenous leukemia, breast cancer, lung cancer, and pancreatic cancer.

Further, the autoimmune disease is selected from systemic lupus erythematosus, type 1 diabetes, rheumatoid arthritis, multiple sclerosis, ankylosing spondylitis, psoriasis, coeliac disease, ulcerative colitis, and Crohn's disease.

Further, the allergic disease is selected from allergic dermatitis, allergic conjunctivitis, allergic asthma, and allergic rhinitis.

In some embodiments of the present application, the allergic disease being canine and feline allergic dermatitis, which having one or more of the following symptoms: cutaneous pruritus, rash, hair loss, desquamation, edema, ulcer, and the like, sometimes accompanied by otitis, sneezing, tearing, and other symptoms, particularly skin itching. In one embodiment of the present application, the disease is canine allergic cutaneous pruritus.

The present application has the following technical effects.

Based on the previous research results of the inventors, in the present application, after experimental research, the free crystal form and the hydrochloride crystal form of the compound represented by formula I are further obtained. The crystal forms have sharp diffraction peaks, high crystallinity, less solvent residue, a single DSC thermal signal, small TGA weight loss, and a high ligand safety level. It can be seen that they all have high solid-state stability and fluidity, low hygroscopicity, and high solubility, which are conductive to preparation and storage of a pharmaceutical preparation. Therefore, they have very high application value and drug development prospects. In addition, clinical trial results show that the free crystal form and the hydrochloride crystal form of the present application have very good pharmacokinetic properties, and their systemic exposure increases linearly in a dose-related manner in experimental animals, the plasma concentration reaches a peak quickly, the in vivo stability is high, there is no obvious drug accumulation after continuous administration, the clinical toxicity is low, the safety is high, and they are significantly better than a positive control drug. Furthermore, the free crystal form and the hydrochloride crystal form of the present application have high oral bioavailability and have good therapeutic effects on various diseases, such as good inhibitory and antipruritic effects on canine allergic pruritus symptoms. In addition, the free crystal form and the hydrochloride crystal form of the present application may be administered through various routes of administration (such as injection and oral administration) and may be prepared into various preparation forms, and have very good application prospects.

### BRIEF DESCRIPTION OF THE DRAWINGS

FIG. 1 shows an X-Ray powder diffraction (XRPD) pattern of a free crystal form A;
FIG. 2 shows a thermogravimetric analysis (TGA)/differential scanning calorimetry (DSC) pattern of a free crystal form A;
FIG. 3 shows a proton nuclear magnetic resonance (¹H NMR) spectrum of a free crystal form A;
FIG. 4 shows an XRPD pattern of a hydrochloride crystal form A;
FIG. 5 shows a TGA/DSC pattern of a hydrochloride crystal form A;
FIG. 6 shows an ¹H NMR spectrum of a hydrochloride crystal form A;
FIG. 7 shows an XRPD pattern of a sulfate crystal form A;
FIG. 8 shows a TGA/DSC pattern of a sulfate crystal form A;
FIG. 9 shows an ¹H NMR spectrum of a sulfate crystal form A;
FIG. 10 shows an XRPD pattern of a phosphate crystal form A;
FIG. 11 shows a TGA/DSC pattern of a phosphate crystal form A;
FIG. 12 shows an ¹H NMR spectrum of a phosphate crystal form A;
FIG. 13 shows an XRPD pattern of a sample of a free crystal form A dissolved in H₂O;
FIG. 14 shows an XRPD pattern of a sample of a free crystal form A dissolved in simulated gastric fluid (SGF);
FIG. 15 shows an XRPD pattern of a free crystal form A in fasted state simulated intestinal fluid (FaSSIF);
FIG. 16 shows an XRPD pattern of a sample of a free crystal form A dissolved in fed state simulated intestinal fluid (FeSSIF);
FIG. 17 shows an XRPD pattern of a sample of a hydrochloride crystal form A dissolved in a pH buffer;
FIG. 18 shows an XRPD pattern of a sample of a free crystal form A dissolved in a pH buffer;
FIG. 19 shows a dynamic vapor sorption (DVS) isotherm of a hydrochloride crystal form A;
FIG. 20 shows a DVS isotherm of a free crystal form A;
FIG. 21 shows overlaid of XRPD patterns of a hydrochloride crystal form A before and after DVS testing;
FIG. 22 shows overlaid of XRPD patterns of a free crystal form A before and after DVS testing;
FIG. 23 shows overlaid of XRPD patterns of a hydrochloride crystal form A before and after stability assessment;
FIG. 24 shows overlaid of XRPD patterns of a free crystal form A before and after stability assessment;
FIG. 25 shows an ultra-performance liquid chromatography (UPLC) chromatogram for stability assessment of a hydrochloride crystal form A (note: a main peak time deviation may be caused by a change in system pressure when the tests are performed at different time points. During each test, a starting sample is tested to determine Application Programming Interface (API) peak time);
FIG. 26 shows a UPLC chromatogram for stability assessment of a free crystal form A (note: a main peak time deviation may be caused by a change in system pressure when the tests are performed at different time points. During each test, a starting sample is tested to determine Application Programming Interface (API) peak time);
FIG. 27 shows a polarized light microscopy (PLM) image of a hydrochloride crystal form A;
FIG. 28 shows a PLM image of a free crystal form A;
FIG. 29 shows XRPD patterns of a hydrochloride crystal form A before and after grinding and tableting;
FIG. 30 shows XRPD patterns of a free crystal form A before and after grinding and tableting;
FIG. 31 shows a plasma concentration-time curve (logarithmic coordinates) of a Beagle dog after intravenous injection with 2 mg/kg free crystal form;
FIG. 32 shows plasma concentration-time curves of Beagle dogs after intragastric administration of different doses of free crystal form A;
FIG. 33 shows plasma concentration-time curves of Beagle dogs after the first intragastric administration and the seventh intragastric administration of 6 mg/kg free crystal form A during multiple administration; and
FIG. 34 shows a plasma concentration-time curve of a male Beagle dog after intragastric administration of 6 mg/kg hydrochloride crystal form A.

### DETAILED DESCRIPTION

Unless otherwise defined, all scientific and technical terms used in the present application have the same meanings as commonly understood by those of ordinary skill in the art to which the present application relates.

In the present application, the term "crystal form" is verified and characterized by an X-ray powder diffraction (XRPD) pattern. Those skilled in the art will appreciate that the physicochemical properties discussed herein can be characterized, with experimental errors depending on instrument conditions, sample preparation, sample purity, and the like. In particular, it is well known to those skilled in the art that XRPD patterns generally vary depending on instrument conditions. It is particularly important to point out that the relative intensity of the XRPD pattern may also change with changes in experimental conditions, so the order of peak intensity cannot be taken as the only or decisive factor. In fact, the relative intensity of the diffraction peaks in the XRPD pattern is related to the preferred orientation of the crystal, and the peak intensities herein are illustrative rather than for absolute comparison. In addition, the experimental error in the peak angles is usually 5% or less, and the error in these angles should also be taken into account, with a typical allowance of ±0.2°. In addition, due to the influence of experimental factors such as sample thickness, the overall shift of the peak angle will be caused, and a certain shift is usually allowed. Therefore, those skilled in the art may appreciate that an XRPD pattern of a crystal form in the present application is not necessarily completely consistent with an XRPD in the embodiments referred to herein, and the "same XRPD pattern" mentioned herein does not mean absolutely identical, the same peak position may differ by ±0.2° and the peak intensity is allowed to have a certain variability. Any crystal form having the same or similar characteristic peaks as those in these patterns falls within the scope of the present application. Those skilled in the art may compare the pattern listed in the present application with a pattern of an unknown crystal form to determine whether the two patterns reflect the same or different crystal forms. In some embodiments, the crystal form A of the present application is pure and single, and is substantially not mixed with any other crystal forms. In the present application, "substantially not", when used to refer to a new crystal form, means that this crystal form contains less than 20% (by weight) of other crystal forms, especially less than 10% (by weight) of other crystal forms, more preferably less than 5% (by weight) of other crystal forms, and even more preferably less than 1% (by weight) of other crystal forms.

It should be noted that the numerical values and numerical ranges mentioned in the present application should not be narrowly understood as the numerical values or numerical ranges themselves. Those skilled in the art should understand that the specific numerical values may slightly fluctuate according to different specific technical environments without departing from the spirit and principles of the present application. In the present application, such fluctuating ranges that may be foreseen by those skilled in the art are often expressed by the term "approximately". When the term "approximately" is used before a numerical value in the present application and refers to the numerical value, it means any value within the range of ±10%, preferably within the range of ±5%, more preferably within the range of ±2%, and even more preferably within the range of ±1% of the value. For example, "approximately 10" should be interpreted as meaning 9 to 11, preferably 9.5 to 10.5, more preferably 9.8 to 10.2, and even more preferably 9.9 to 10.1.

It should also be noted that in the X-ray diffraction patterns of powder samples, the diffraction pattern obtained from the crystalline compound is often characteristic of a specific crystal form, and the relative intensity of the diffraction peaks (especially at low angles) may vary due to the dominant orientation effect caused by differences in crystallization conditions, particle size, relative content of the mixture, and other test conditions. Therefore, the relative intensity of the diffraction peaks is not characteristic of the crystal in question. During determination of whether it is the same as a known crystal form, more attention should be paid to the position of the peaks rather than the relative intensity of the peaks.

In the present application, the term "room temperature" means that the temperature of an object is close to or the same as the temperature of a space (such as a place of a fume hood in which the object is located). Typically, room temperature is from approximately 20°C to approximately 30°C, or from approximately 22°C to 27°C, or approximately 25°C.

Antisolvent crystallization (also known as antisolvent addition, precipitation crystallization, salting out or forced crystallization) is a method in which one or more antisolvents are added to a solution in which a target product is dissolved in a positive solvent, and the product is slightly soluble in the solution, so that the solution reaches a supersaturated state and crystals are precipitated. Anti-antisolvent crystallization is a method in which after a target product is usually dissolved in a positive solvent to obtain a solution, the solution is added to one or more antisolvents, and the product is slightly soluble in the solution, so that the solution reaches a supersaturated state and crystals are precipitated.

The ability of the antisolvent to dissolve the target product is worse than that of the positive solvent, for example, the difference is more than 10%, 20%, 30%, 40%, 50%, 60%, 70%, or 80%, so the antisolvent in the system is relative. The positive solvent and the antisolvent may be polar solvents or nonpolar solvents, for example, may be selected from one or more of dimethylformamide (DMF), dimethyl sulfoxide (DMSO), water, an alcohol solvent, an ether solvent, a ketone solvent, an ester solvent, an alkane solvent, an aromatic solvent, and a nitrile solvent. The alcohol solvent includes, but is not limited to, methanol, ethanol, propanol, isopropanol, 1,3-propanediol, 1,2-propanediol, chlorobutanol, or a combination thereof. The ether solvent includes, but is not limited to, tetrahydrofuran, methyl tert-butyl ether, 1,4-dioxane, or a combination thereof. The ketone solvent includes, but is not limited to, acetone, methyl ethyl ketone, 4-methyl-2-pentanone, or a combination thereof. The ester solvent includes, but is not limited to, ethyl acetate, isopropyl acetate, n-butyl acetate, tert-butyl acetate, or a combination thereof. The alkane solvent includes, but is not limited to, dichloromethane, chloroform, n-hexane, cyclohexane, pentane, n-heptane, or a combination thereof. The aromatic solvent includes, but is not limited to, benzene, toluene, or a combination thereof. The nitrile solvent includes, but is not limited to, acetonitrile and malononitrile.

Antisolvent crystallization, anti-antisolvent crystallization may be performed by batch, semi-batch, or continuous crystallization. The antisolvent is added to the solution (antisolvent crystallization) or the product solution is added to the antisolvent (anti-antisolvent crystallization), and the addition may be performed dropwise at a constant rate, or slowly at the beginning and then at a gradually increasing rate.

In the present application, unless otherwise specified, the "animal" described herein is a non-human animal, in particular a mammal, such as a monkey, a pig, a cow, a sheep, a horse, a donkey, a dog, a cat, a rabbit, a mouse, a fox, a raccoon dog, a mink, and a camel, etc. In some embodiments of the present application, the animal is a pet, such as a dog, a cat, a rodent (such as a chinchilla, a pet rabbit, a hamster, a guinea pig, a mouse, a gerbil, a Siberian chipmunk, a squirrel, a complex-toothed flying squirrel, a chipmunk, a reverse-coated guinea pig, and a degu, etc).

The term "treating" refers to preventing, curing, reversing, attenuating, alleviating, minimizing, inhibiting, suppressing and/or halting one or more clinical symptoms of a disease after onset of the disease.

The term "preventing" refers to avoiding, minimizing or making the onset or development of a disease difficult by treating the disease before onset of the disease.

The term "inflammation" is the defensive response of the body to stimulation, manifested as redness, swelling, heat, pain, dysfunction, and the like. It may be infectious inflammation caused by infection, or non-infectious inflammation not caused by infection, such as inflammation caused by immune response (such as various types of hypersensitivity reactions, or inflammation caused by some autoimmune diseases). The term "inflammatory disease" refers to a disease characterized by inflammation.

The term "tumor" refers to an abnormal mass of tissue in which the growth of the mass exceeds and is not coordinated with the growth of normal tissue. Tumors may be either "benign" or "malignant" depending on characteristics such as degree of cell differentiation (both morphological and functional), growth rate, local invasion, and metastasis. "Benign tumors" are generally well-differentiated and are characterized by growing slower than malignant tumors and remaining confined to the site of origin. In addition, benign tumors do not have the ability to infiltrate, invade, or metastasize to distant sites. In some cases, certain "benign" tumors may later give rise to malignant tumors, possibly due to additional genetic changes in a subpopulation of neoplastic cells of the tumors, and these tumors are called "precancerous tumors". "Malignant tumors" are usually poorly differentiated (anaplastic) and have a characteristic rapid growth pattern with progressive infiltration, invasion, and destruction of surrounding tissues. In addition, malignant tumors often have the ability to metastasize to distant sites.

The term "cancer" refers to a malignant tumor (Stedman's Medical Dictionary, 25th ed.; Hensyl ed.; Williams & Wilkins: Philadelphia, 1990).

The term "autoimmune disease" refers to a disease caused by the immune response of the body to antigens in the body, resulting in damage to tissues of the body. The American Autoimmune Related Diseases Association has published a comprehensive list of autoimmune diseases.

Various publications, patents, and published patent descriptions are cited herein, the disclosures of which are incorporated by reference in their entireties.

The embodiments of the present application will be described in detail below with reference to examples, but those skilled in the art will appreciate that the following examples are only used to illustrate the present application and should not be construed as limiting the scope of the present application. If no specific conditions are specified in the examples, the experiments are carried out according to conventional conditions or conditions recommended by the manufacturer. The reagents or instruments used without indicating the manufacturer are all conventional products that are commercially available.

The following solvents are used in the examples:
methanol (MeOH); ethanol (EtOH); isopropyl alcohol (IPA); n-butyl alcohol (n-BuOH); acetone; methyl isobutyl ketone (MIBK); 2-butanone (MEK); ethyl acetate (EtOAc); isopropyl acetate (IPAc); n-butyl acetate; methyl tert-butyl ether (MTBE); cyclopentyl methyl ether (CPME); tetrahydrofuran (THF); 2-methyltetrahydrofuran (2-MeTHF); 1,4-dioxane; acetonitrile (ACN); dichloromethane (DCM); toluene; n-heptane; dimethyl sulfoxide (DMSO); anisole; cyclohexane; and n-hexane.

A structure of a compound A in the examples is as follow:

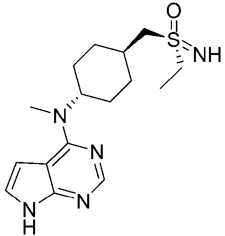

A preparation method of the compound A included the following steps.

### Step 1: Synthesis of compound 2

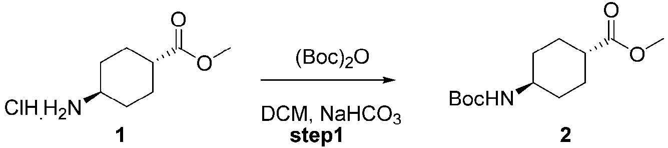

At room temperature, a compound 1 (500 g, 2.58 mol), dichloromethane (1 L), and purified water (1 L) were sequentially placed into a 5 L three-necked flask, mechanical stirring was started, sodium bicarbonate (400 g, 4.76 mol) and di-tert-butyl dicarbonate (650 g, 2.98 mol) were sequentially added, the mixture was stirred at room temperature for 4 h after addition was completed, dichloromethane (1 L) and purified water (1 L) were added, phases were separated, an aqueous phase was extracted with aqueous phase (800 mL × 2), organic phases were combined, washed with saturated brine (1000 mL × 1), dried over anhydrous sodium sulfate, filtered, and concentrated under reduced pressure at 40°C until no distillate was observed, an obtained substance was triturated with petroleum ether (500 mL) at room temperature for 0.5 h, filtered under reduced pressure, and a filter cake was collected and dried under forced air at 25°C for 16 h to obtain a white solid (513 g, yield=77%).

### Step 2: Synthesis of compound 3

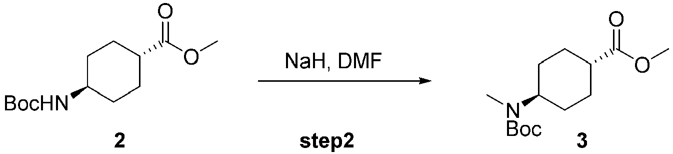

At room temperature, the compound 2 (513 g, 2.58 mol) and anhydrous DMF (2 L) were sequentially placed into a 5 L three-necked flask, mechanical stirring was started, the mixture was purged with nitrogen via three vacuum-refill cycles, the mixture was cooled to 10°C in an ice bath, sodium hydride (103.8 g, 2.59 mol) was added portionwise with negligible exotherm, the mixture was stirred for 0.5 h after addition was completed, and heated to room temperature in a warm water bath, iodomethane (340 g, 2.40 mol) was added dropwise within 100 min with negligible exotherm, upon the mixture was slowly heated to 36°C to 38°C, a violent exothermic reaction occurred (uncontrolled heat evolution), and then the mixture was cooled to room temperature and stirred overnight. The reaction solution was slowly poured into a saturated NH₄Cl solution (2 L) while the temperature was maintained below 20°C, and then extracted with EA (1 L × 3), an organic phase was washed with saturated NaCl (500 mL × 3), and concentrated to obtain an oily substance (550 g), and the oily substance was purified by flash column chromatography (eluent: petroleum ether/ethyl acetate = 10/1 to 5/1) to obtain a colorless oily substance (517 g, yield=95.9%).

### Step 3: Synthesis of compound 4

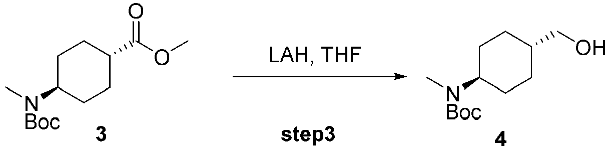

Anhydrous tetrahydrofuran (300 mL) was placed into a 5 L three-necked flask, cooled to 10°C to 15°C in an ice bath, stirring was started under N₂ atmosphere, lithium aluminium hydride (46 g, 1.21 mol) was added portionwise while the temperature was maintained below -5°C, a solution of compound 3 (516 g, 1.90 mol) in anhydrous tetrahydrofuran (1.5 L) was added dropwise, the mixture reacted for 2.5 h after addition was completed, a 15% sodium hydroxide solution (46 mL) and water (135 mL) were added dropwise while the temperature was maintained at 0°C to 10°C, the mixture was filtered under reduced pressure, a filter cake was rinsed with ethyl acetate (500 mL), a filtrate was collected after filtration was completed, ethyl acetate (500 mL) and water (1000 mL) were respectively added to the filtrate, phases were separated, an organic phase was collected, an aqueous phase was extracted with EA (500 mL × 3), and organic phases were combined, washed with a saturated sodium chloride solution (500 mL × 3), dried, and concentrated to obtain colorless oily substance (407 g, yield=88.1%).

### Step 4: Synthesis of compound 5

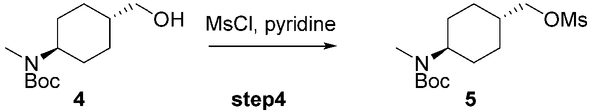

At room temperature, the compound 4 (407 mg, 1.67 mol), anhydrous dichloromethane (1000 mL), and pyridine (304.3 g, 3.9 mol) were sequentially placed into a 3 L three-necked flask, stirring was started under nitrogen atmosphere, methylsufonyl chloride (250 g, 2.18 mol) was added dropwise while the solution was cooled to 10°C to 15°C in an ice bath, the mixture was stirred at room temperature overnight after addition was completed, water (500 mL) was added to quench the reaction, and an aqueous phase was extracted with dichloromethane (500 mL × 3), washed with a saturated NaCl solution (1000 mL × 1), dried, and concentrated to obtain a yellow oily substance (510.2 g, yield=94.5%).

### Step 5: Synthesis of compound 6

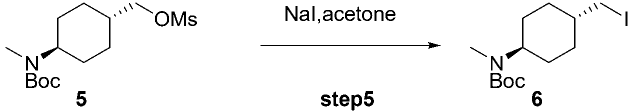

At room temperature, the compound 5 (510 g, 1.59 mol), acetone (3000 mL), and NaI (310 g, 2.07 mol) were sequentially placed into a 10 L three-necked flask, the mixture was stirred, heated to reflux overnight, and cooled to room temperature, water (1000 mL) was added to quench the reaction, and the reaction solution was extracted with ethyl acetate (1 L × 3), washed with saturated NaCl (2 L), dried, and concentrated to obtain an oily substance (423 g, yield=75.8%), which was used directly in the next step.

### Step 6: Synthesis of compound 8

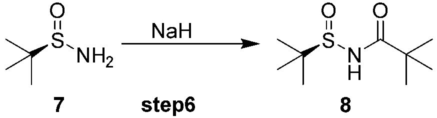

At room temperature, the compound 7 (35 g, 0.289 mol) and anhydrous tetrahydrofuran (1100 mL) were placed into a 5 L three-necked flask, the mixture was stirred and cooled to 0°C to 5°C, sodium hydride (27.5 g, 0.687 mol) was added portionwise with vigorous exotherm and then a solution of pivalic anhydride (51.2 g, 0.275 mol) in THF (1000 mL) was added dropwise while the temperature was maintained below 20°C, the mixture reacted at room temperature for 2 h, methanol (100 mL) was added to the system, an aqueous NH₄Cl solution (600 mL) and saturated brine (600 mL) were added dropwise to quench the reaction while the temperature was maintained at 0°C to 20°C, the reaction solution was extracted with ethyl acetate (500 mL × 3), an organic phase was washed with saturated NaCl (1 L), dried, and concentrated to obtain an oily substance, the oily substance was purified by column chromatography (eluent: PE/EA=5/1, 3/1) to obtain a solid, and the solid was triturated with petroleum ether (50 mL) to obtain a white solid (45.8 g, yield=81.3%).

### Step 7: Synthesis of compound 9

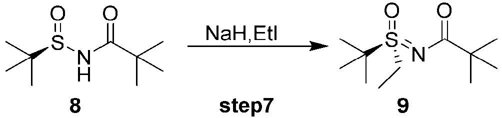

At room temperature, the compound 8 (45 g, 0.22 mol), 1,4-dioxane (200 mL), and 15-crown-5 (58 g) were sequentially placed into a 500 mL three-necked flask, the mixture was stirred and cooled to 10°C to 20°C, sodium hydride (10.5 g, 0.26 mol) was added portionwise and then ethyl iodide (68.4 g, 1.44 mol) was added dropwise at room temperature, the mixture was stirred at 40°C overnight after addition was completed, an aqueous ammonium chloride solution (100 mL) was added to the system to quench the reaction, an aqueous phase was extracted with ethyl acetate (100 mL × 3), washed with saturated NaCl (200 mL), dried, and concentrated to obtain an oily substance, and the oily substance was purified by column chromatography (eluent: PE/EA=10/1, 7/1, 5/1, 3/1) to obtain a white solid (45.26 g, yield=88.3%).

### Step 8: Synthesis of compound 10

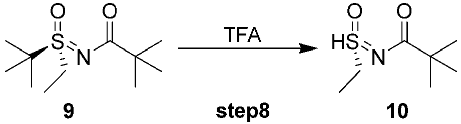

At room temperature, the compound 9 (45 g, 0.19 mol) and dichloromethane (200 mL) were placed into a 1 L three-necked flask, the mixture was stirred, trifluoroacetic acid (33 g, 0.29 mol) was added at room temperature, the mixture was stirred at room temperature for 3 h after addition was completed, a saturated aqueous sodium chloride solution (100 mL) was add to the system to quench the reaction, the reaction solution was extracted with dichloromethane (50 mL × 3), washed with saturated sodium bicarbonate (100 mL), dried, and concentrated to obtain a solid crude product, and the solid crude product was purified by column chromatography (eluent: PE/EA=5/1, 3/1, 1/1) to obtain a white solid (27 g, yield=80.2%).

### Step 9: Synthesis of compound 11

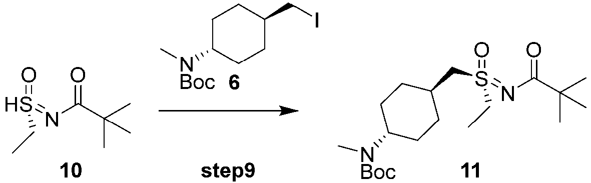

At room temperature, the compound 10 (26 g, 0.146 mol), 1,4-dioxane (146 mL), and 15-crown-5 (48.5 g) were sequentially placed into a 500 mL three-necked flask, the mixture was stirred and cooled to 10°C to 20°C, sodium hydride (8.8 g, 0.22 mol) was added portionwise and then a solution of the compound 6 (103.8 g, 0.293 mol) in THF (50 mL) was added dropwise at room temperature, the mixture was stirred at 50°C for 5 d after addition was completed, an aqueous ammonium chloride solution (100 mL) was added to the system to quench the reaction, the reaction was extracted with EA (100 mL × 3), an organic phase was washed with saturated NaCl (200 mL), dried, and concentrated to obtain an oily substance, and the oily substance was purified by column chromatography (eluent: PE/EA=10/1, 7/1, 5/1, 3/1) to obtain a white solid (31.4 g, yield=53.5%).

### Step 10: Synthesis of compound 12

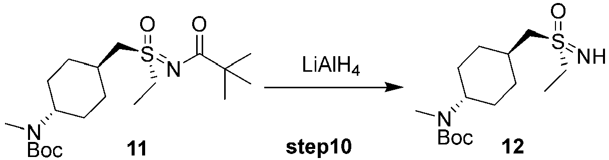

The compound 11 (31 g, 0.077 mol) and anhydrous tetrahydrofuran (500 mL) were placed into a 1 L three-necked flask, the solution was cooled to 10°C in an ice bath, the stirring was started under N₂ atmosphere, lithium aluminium hydride (8.8 g, 0.24 mol) was added portionwise, the mixture reacted for 1 h after addition was completed, a saturated sodium sulfate solution (24 mL) was added dropwise to quench the reaction while the temperature was maintained at 0°C to 10°C, the reaction solution was diluted with ethyl acetate (200 mL) and filtered under reduced pressure, a filter cake was rinsed with ethyl acetate (50 mL), a filtrate was collected after filtration was completed, water (100 mL) was added, phases were separated, an aqueous phase was extracted with ethyl acetate (50 mL × 2), organic phases were combined and washed with a saturated sodium chloride solution (100 mL × 1), dried, and concentrated to obtain a colorless oily substance, and the colorless oily substance was purified by column chromatography (eluent: ethyl acetate) to obtain an oily substance (18.5 g, yield=80%).

### Step 11: Synthesis of compound 13

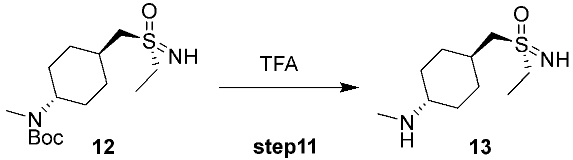

At room temperature, the compound 12 (18.5 g, 0.058 mol) and dichloromethane (45 mL) were placed into a 250 mL three-necked flask, the mixture was stirred, trifluoroacetic acid (20 mL) was added at room temperature, the mixture was stirred at room temperature overnight after addition was completed, and concentrated until no distillate was observed to obtain a crude product (13.3 g), which was used directly in the next step.

### Step 12: Synthesis of compound 15

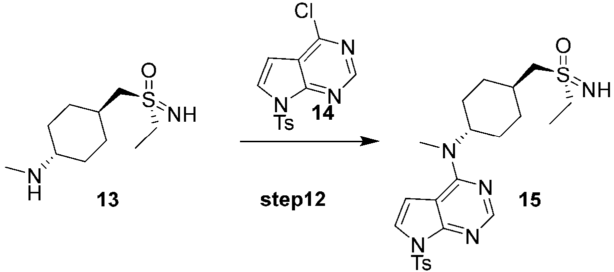

The compound 13 (13.3 g, 0.058 mol), anhydrous DMF (100 mL), potassium carbonate (33.4 g, 0.24 mol), and the compound 14 (18.7 g, 0.058 mol) were sequentially placed into a 250 mL three-necked flask, the mixture was stirred, heated to 115°C, reacted overnight, and cooled to room temperature, water (150 mL) was added to quench the reaction, the reaction solution was extracted with ethyl acetate (100 mL × 6) and washed with saturated NaCl (500 mL × 3), an organic phase was dried and concentrated to obtain an oily substance, the oily substance was purified by column chromatography (eluent: PE/EA=1/1, DCM/MeOH=25/1) to obtain a semi-solid product, and the semi-solid product was triturated with ethyl acetate (10 mL) and n-hexane (50 mL) to obtain an off-white solid (22.4 g, yield=76.5%).

### Step 13: Synthesis of compound A

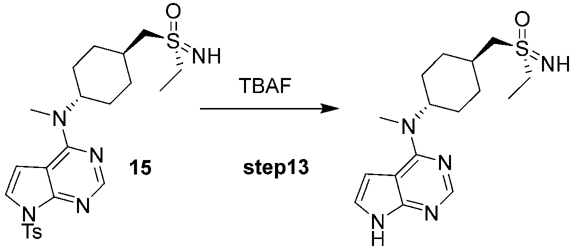

The compound 15 (22.4 g, 0.045 mol), anhydrous THF (250 mL), and a TBAF solution (93 mL, 1 M/L) were placed into a 500 mL three-necked flask, the mixture was stirred, heated to reflux overnight, cooled, and concentrated until no distillate was observed, water (150 mL) and ethyl acetate (200 mL) were sequentially added to the system, an organic phase was separated, an aqueous phase was extracted with ethyl acetate (100 mL × 3), washed with saturated NaCl (500 mL × 1), dried over anhydrous sodium sulfate, and concentrated to obtain a solid crude product, and the solid product was purified by column chromatography (eluent: DCM/MeOH=25/1), and triturated with ethyl acetate (20 mL) to obtain a white solid (11 g, yield=73.3%).

Ms: 336.2 [M+H]⁺. ¹H NMR (400 MHz, DMSO) δ 11.60 (s, 1H), 8.08 (s, 1H), 7.11 (s, 1H), 6.53 (s, 1H), 4.67 (s, 1H), 3.57 (s, 1H), 3.16 (s, 3H), 2.97 (q, *J*=7.6 Hz, 2H), 2.93(d, *J*=6.0 Hz, 2H), 2.12-1.99 (m, 3H), 1.75-1.68 (m, 4H), 1.33-1.24 (m, 2H), 1.22 (t, *J*=7.6 Hz, 3H).

### Example 1 Preparation and characterization of free crystal form of compound A

### 1.1 Instruments and methods

### 1.1.1 XRPD

XRPD results were collected on a PANalytical X'Pert³ and Empyrean X-ray powder diffractometer. Samples were placed in the center of a background-free silicon wafer for testing. Test parameters are summarized in Table 1.

**Table 1 XRPD testing parameters**

| **Instrument model** | **X' Pert³ *&* Empyrean** |
|---|---|
| X-ray | Cu, kα; Kα1 (Å): 1.540598, |
| | Kα2 (Å): 1.544426 |
| | Kα2/Kα1 intensity ratio: 0.50 |
| X-ray tube settings | 45 kV, 40 mA |
| Divergence slit | 1/8° |
| Scan mode | Continuous |
| Scan range (°2TH) | 3° to 40° |
| Step size (°2TH) | 0.0263 |
| Dwell time per step (s) | 46.665 |
| Scan duration (s) | Up to 5 min 04 s |

### 1.1.2 Thermogravimetric analysis (TGA) and differential scanning calorimetry (DSC)

TGA and DSC patterns were collected on a TA Q5000 thermogravimetric analyzer and a TA Discovery 2500 differential scanning calorimeter, respectively. Test parameters are summarized in Table 2.

**Table 2 TGA and DSC testing parameters**

| **Parameter** | **TGA** | **DSC** |
|---|---|---|
| Method | Linear heating | Linear heating |
| Sample crucible | Aluminum crucible with opening | Aluminum crucible with crimped lid |
| Temperature range | Room temperature to 320°C | 25°C to 290°C |
| Heating rate | 10°C/min | 10°C/min |
| Protective gas | Nitrogen | Nitrogen |

### 1.1.3 Proton nuclear magnetic resonance (¹H NMR)

¹H NMR spectra were collected on a Bruker 400M NMR spectrometer using DMSO-*d*₆ as a solvent.

### 1.1.4 Dynamic vapor sorption (DVS)

DVS isotherms were collected on an SMS (Surface Measurement Systems) DVS Intrinsic. Relative humidity at 25°C was calibrated using the deliquescence points of LiCl, Mg(NO₃)₂, and KCl. Test parameters are summarized in Table 3.

**Table 3 DVS testing parameters**

| **Parameter** | **DVS** |
|---|---|
| Temperature | 25°C |
| Sample mass | 20 mg to 40 mg |
| Protective gas and flow rate | N₂, 200 mL/min |
| dm/dt | 0.002%/min |
| Minimum dm/dt equilibration time | 10 min |
| Maximum equilibration time | 180 min |
| RH range | 0% RH to 95% RH to 0% RH |
| RH gradient | 10% RH (0% RH to 90% RH & 90% RH to 0% RH) |
| | 5% RH (90% RH to 95% RH & 95% RH to 90% RH) |

### 1.1.5 Ultra-performance liquid chromatography (UPLC)

In the tests, the purity was tested by using a Waters H-Class UPLC system. Analysis conditions are summarized in Table 4.

**Table 4 UPLC testing conditions**

| **UPLC** | **Waters H-Class UPLC (PDA detector)** | |
|---|---|---|
| Chromatographic column | ACQUITY UPLC HSS T3, 100×2.1 mm, 1.8 µm | |
| Mobile phase | A: 0.05% TFA in H₂O | |
| | B: 0.05% TFA in ACN | |
| | Time (min) | %B |
| | 0.00 | 0 |
| | 9.00 | 30 |
| Elution gradient | 11.00 | 95 |
| | 12.00 | 95 |
| | 12.01 | 0 |
| | 13.00 | 0 |
| Run time | 13.0 min | |
| Post-run time | 0.0 min | |
| Mobile phase flow rate | 0.6 mL/min | |
| Injection volume | 2 µL | |
| Detection wavelength | UV at 214 nm | |
| Column temperature | 45°C | |
| Injector temperature | RT | |

### 1.2 Preparation and characterization of free crystal form A of compound A

A free crystal form A was obtained by an antisolvent addition method using MeOH as a good solvent and MIBK as an antisolvent (Test No. 02-A1 shown in Table 6).

An XRPD pattern of the free crystal form A is shown in FIG. 1, and XRPD diffraction peak data are summarized in Table 5.

TGA/DSC patterns of the free crystal form A are shown in FIG. 2. The TGA results demonstrate that the free crystal form A has a weight loss of 1.7% when heated from room temperature to 180°C. The DSC results reveal one sharp endothermic peak at 197.4°C (starting temperature), which is presumed to be a melting signal.

An ¹H NMR spectrum of the free crystal form A is shown in FIG. 3.

The foregoing characterization results demonstrate that the free crystal form A of the compound A has a small TGA weight loss and a single DSC signal. Therefore, it is speculated that the free crystal form A is an anhydrous crystal form.

**Table 5 XRPD diffraction peak data of free crystal form A**

| **2θ [°]** | **Interplanar spacing [Å]** | **Relative intensity [%]** |
|---|---|---|
| 4.236 | 20.85989 | 7.73 |
| 12.393 | 7.14212 | 34.21 |
| 13.241 | 6.68684 | 16.39 |
| 14.616 | 6.06082 | 100.00 |
| 15.231 | 5.81746 | 12.28 |
| 15.545 | 5.70039 | 4.13 |
| 16.652 | 5.32396 | 39.41 |
| 17.192 | 5.15803 | 70.63 |
| 17.925 | 4.94850 | 9.28 |
| 19.289 | 4.60170 | 19.80 |
| 20.318 | 4.37080 | 49.40 |
| 21.222 | 4.18673 | 21.77 |
| 21.561 | 4.12156 | 14.15 |
| 23.913 | 3.72137 | 18.04 |
| 24.817 | 3.58771 | 35.81 |
| 25.035 | 3.55703 | 37.11 |
| 25.445 | 3.50059 | 5.73 |
| 26.985 | 3.30427 | 16.89 |
| 27.431 | 3.25152 | 9.54 |
| 28.136 | 3.17168 | 5.63 |
| 28.477 | 3.13439 | 6.60 |
| 30.396 | 2.94074 | 5.50 |
| 31.772 | 2.81652 | 3.35 |
| 33.590 | 2.66812 | 6.41 |
| 34.452 | 2.60326 | 2.72 |
| 36.316 | 2.47379 | 7.86 |
| 37.274 | 2.41243 | 2.12 |
| 38.092 | 2.36244 | 1.69 |

A specific experimental method and results of preparation of the foregoing crystal form were as follows:
approximately 20 mg of compound A sample was weighed and placed into a 20 mL vial, 0.3 mL to 1.0 mL of good solvent (see Table 6) was added to dissolve the sample, the antisolvent in Table 6 was added dropwise to the clear solution while the solution was stirred until a solid was precipitated, and if no solid was precipitated after 5 mL of antisolvent was added, the antisolvent was no longer added. The precipitated solid was centrifuged and separated, and subjected to XRPD testing. If no solid was precipitated, the solution was stirred at 5°C or volatilized at room temperature. Test results are summarized in Table 6. The free crystal form A and an oil sample were obtained in the antisolvent addition test.

**Table 6 Test results**

| **Test No.** | **Good solvent** | **Antisolvent** | **Result** |
|---|---|---|---|
| 02-A1^{&} | MeOH | MIBK | Free crystal form A |
| 02-A2^{#} | | IPAc | Free crystal form A |
| 02-A3 | | MTBE | Free crystal form A |
| 02-A4* | 1,4-Dioxane | n-Heptane | Free crystal form A |
| 02-AS^{&} | | EtOAc | Free crystal form A |
| 02-A6^{&} | | MEK | Oil |
| 02-A7 | CHCl₃ | n-Heptane | Free crystal form A |
| 02-A8 | | m-Xylene | Free crystal form A |
| 02-A9 | | CPME | Free crystal form A |
| 02-A10* | DMSO | Toluene | Free crystal form A |
| 02-A11^{&} | | Anisole | Free crystal form A |
| 02-A12^{&} | | H₂O | Free crystal form A |

| | | | |
|---|---|---|---|
| *: After the antisolvent was added, a clear solution or oily sample was obtained, and a solid was precipitated by stirring at room temperature overnight. ^{#}: After the antisolvent was added, a clear solution was obtained, which remained clear after stirring at room temperature overnight, and a solid was precipitated after the solution was stirred at 5°C for 7 d. ^{&}: After the antisolvent was added, a clear solution was obtained, which remained clear after stirring at room temperature/5°C, and a solid was precipitated after the solution was volatilized at room temperature. | | | |

### Example 2 Preparation and characterization of salt crystal form of compound A

### 2.1 Instruments and methods

### 2.1.1 XRPD

XRPD results were collected on a PANalytical X'Pert³ and Empyrean X-ray powder diffractometer. Test parameters are summarized in Table 7.

**Table 7 XRPD testing parameters**

| **Mode** | **Conventional reflection** |
|---|---|
| X-ray | Cu, kα; Kα1 (Å): 1.540598, Kα2 (Å): 1.544426 |
| | Kα2/Kα1 intensity ratio: 0.50 |
| X-ray tube settings | 45 kV, 40 mA |
| Divergence slit | 1/8° |
| Scan mode | Continuous |
| Scan range (°2TH) | 3° to 40° |
| Step size (°2TH) | 0.0263 |
| Dwell time per step (s) | 46.67 |
| Scan duration (s) | 5 min 04 s |

### 2.1.2 TGA and DSC

TGA results were collected on a TA Discovery 5500 thermogravimetric analyzer, and DSC results were collected on a TA Discovery 2500 differential scanning calorimeter. Test parameters are summarized in Table 8.

**Table 8 TGA and DSC testing parameters**

| **Parameter** | **TGA** | **DSC** |
|---|---|---|
| Method | Linear heating | Linear heating |
| Sample crucible | Aluminum crucible with opening | Aluminum crucible with crimped lid |
| Temperature range | Room temperature to 350°C | 25°C to 300°C |
| Heating rate | 10°C/min | 10°C/min |
| Protective gas | Nitrogen | Nitrogen |

### 2.1.3 ¹H NMR

¹H NMR spectra were collected on a Bruker 400M NMR spectrometer using DMSO-*d*₆ as a solvent.

### 2.1.4 DVS

DVS isotherms were collected on an SMS (Surface Measurement Systems) DVS Intrinsic. Relative humidity at 25°C was calibrated using the deliquescence points of LiCl, Mg(NO₃)₂, and KCl. DVS testing parameters are summarized in Table 9.

**Table 9 DVS testing parameters**

| **Parameter** | **DVS** |
|---|---|
| Temperature | 25°C |
| Sample mass | 10-30 mg |
| Protective gas and flow rate | N₂, 200 mL/min |
| dm/dt | 0.002%/min |
| Minimum dm/dt equilibration time | 10 min |
| Maximum equilibration time | 180 min |
| RH range | 0% RH to 95% RH to 0% RH |
| RH gradient | 10% RH (0% RH to 90% RH & 90% RH to 0% RH) |
| | 5% RH (90% RH to 95% RH & 95% RH to 90% RH) |

### 2.1.5 UPLC

In the experiments, the purity, the solubility, and the molar ratio were tested by using a Waters H-Class UPLC system. Analysis conditions are summarized in Table 10.

**Table 10 UPLC testing conditions**

| **UPLC** | **Waters H-Class UPLC** | |
|---|---|---|
| Chromatographic column | ACQUITY UPLC HSS T3, 100×2.1 mm, 1.8 µm | |
| Mobile phase | A: 0.05% TFA in H₂O | |
| | B: 0.05% TFA in ACN | |

| | Time (min) | %B |
|---|---|---|
| Elution gradient | 0.00 | 5 |
| | 9.00 | 30 |
| | 11.00 | 95 |
| | 12.00 | 95 |
| | 12.01 | 5 |
| | 13.00 | 5 |
| Run time | 13.0 min | |
| Post-run time | 0.0 min | |
| Mobile phase flow rate | 0.6 mL/min | |

| **UPLC** | **Waters H-Class UPLC** | |
|---|---|---|
| Injection volume | 2 µL | |
| Detection wavelength | 286 nm | |
| Column temperature | 45°C | |
| Injector temperature | Room temperature | |

### 2.1.6 Polarized light microscopy (PLM)

PLM images were collected on a Zeiss Scope.A1 polarizing microscope.

### 2.1.7 Ion chromatography (IC)

In the experiments, the ion content was analyzed by using a ThermoFisher ICS-1100 IC system. Specific conditions are summarized in Table 11.

**Table 11 IC conditions and parameters**

| **IC** | **ThermoFisher ICS-1100** |
|---|---|
| Chromatographic column | Dionex IonPac AS18 RFIC (4 × 250 mm) |
| Mobile phase | 25 mM NaOH |
| Injection volume | 25 µL |
| Flow rate | 1.0 mL/min |
| Sample compartment temperature | 35°C |
| Column temperature | 35°C |
| Current | 80 mA |
| Run time | 20 min |

### 2.2 Preparation and characterization of salt crystal form of compound A

In this experiment, the free crystal form A of the compound A was taken as a raw material to study the possibility of the free crystal form A of the compound A forming corresponding salt forms in presence of 23 different acids and 4 different solvents. Specific experiments are summarized in Table 12. A specific procedure was as follows: approximately 20 mg of free crystal form A of the compound A and an equimolar amount of corresponding acid ligand were weighed and placed into an HPLC vial, 0.5 mL of solvent was added, the mixture was stirred magnetically (up to 1000 rpm) at room temperature for approximately 4 d, and a solid was separated for XRPD detection. If no solid was precipitated, the mixture was stirred at 5°C/-20°C, added with an antisolvent, or volatilized at room temperature.

The study has found that under 92 different experimental conditions, it is not easy to obtain the corresponding salt crystal form of the free crystal form A of the compound A in the acid system. XRPD comparison indicates that only 3 salt crystal forms may exist stably in 4 different solvents, namely, a hydrochloride crystal form A, a sulfate crystal form A, and a phosphate crystal form A.

**Table 12 Experimental results of salt form screening**

| **Acid** | | **Solvent** | | | |
|---|---|---|---|---|---|
| | | **A: EtOH** | **B: Acetone/H₂O (19:1, v/v)** | **C: EtOAc** | **D: 2-MeTHF** |
| 0 | Blank | Free crystal form A | Free crystal form A | Free crystal form A | Free crystal form A |
| 1 | Hydrochloric acid | Hydrochloride crystal form A | Hydrochloride crystal form A | Hydrochloride crystal form A | Hydrochloride crystal form A^{[1]} |
| 2 | Sulfuric acid | Gel^{[1]} | Sulfate crystal form A | Sulfate crystal form A^{[1]} | Sulfate crystal form A |
| 3 | Phosphoric acid | Free crystal form A | Phosphate crystal form A | Free crystal form A | Free crystal form A |
| 4 | Glutamic acid | Free crystal form A + acid | Free crystal form A + acid | Free crystal form A + acid | Free crystal form A + acid |
| 5 | L-Tartaric acid | Free crystal form A | Gel^{[1]} | Free crystal form A | Free crystal form A |
| 6 | Fumaric acid | Free crystal form A | Free crystal form A | Free crystal form A + peak | Amorphism |
| 7 | Mucic acid | Free crystal form A + acid | Free crystal form A + acid | Free crystal form A + acid | Free crystal form A + acid |
| 8 | Citric acid | Gel^{[1]} | Gel^{[1]} | Free crystal form A | Free crystal form A |
| 9 | Glycolic acid | Free crystal form A | Free crystal form A | Free crystal form A | Free crystal form A |
| 10 | L-Malic acid | Free crystal form A | Free crystal form A | Gel^{[1]} | Free crystal form A |
| 11 | Hippuric acid | Free crystal form A | Free crystal form A | Free crystal form A + acid | Free crystal form A + acid |
| 12 | Gluconic acid | Free crystal form A | Gel^{[1]} | Free crystal form A^{[1]} | Gel^{[1]} |
| 13 | DL-Lactic acid | Free crystal form A | Free crystal form A | Free crystal form A | Free crystal form A |
| 14 | Ascorbic acid | Free crystal form A | Free crystal form A | Free crystal form A + acid | Free crystal form A + acid |
| 15 | Succinic acid | Free crystal form A | Free crystal form A | Free crystal form A + acid | Free crystal form A |
| 16 | Acetic acid | Free crystal form A | Free crystal form A | Free crystal form A | Free crystal form A |
| 17 | 1,5-Naphthalenedi sulfonic acid | Free crystal form A + peak | Free crystal form A + peak | Amorphism | Free crystal form A |
| 18 | p-Toluenesulfon ic acid | Free crystal form A | Gel^{[1]} | Free crystal form A + acid | Free crystal form A + acid |
| 19 | Methanesulfo nic acid | Free crystal form A^{[2]} | Gel^{[1]} | Free crystal form A^{[1]} | Gel^{[1]} |
| 20 | Benzenesulfon ic acid | Amorphism^{[2]} | Gel^{[1]} | Free crystal form A^{[1]} | Free crystal form A^{[1]} |
| 21 | Ethanesulfoni c acid | Free crystal form A^{[2]} | Free crystal form A^{[3]} | Free crystal form A^{[1]} | Free crystal form A + peak^{[1]} |
| 22 | Malonic acid | Free crystal form A | Free crystal form A | Free crystal form A^{[1]} | Free crystal form A |
| 23 | Benzoic acid | Free crystal form A | Free crystal form A | Free crystal form A | Free crystal form A |

| | | | | | |
|---|---|---|---|---|---|
| ^{[1]}: After stirring at room temperature, the solution formed into oil or gel, after cyclical stirring at 50°C to 5°C, the solution still formed into oil or gel, and the solution was vacuum-dried at room temperature. ^{[2]}: After stirring at room temperature, the solution became clear, after stirring at 5°C and -20°C, the solution still remained clear, after addition of the antisolvent n-heptane, the solution formed into oil, and after cyclical stirring at 50°C to 5°C, a solid was precipitated. ^{[3]}: After stirring at room temperature, the solution became clear, after stirring at 5°C and -20°C, the solution remained clear, after addition of the antisolvent n-heptane, the solution formed into oil, after cyclical stirring at 50°C to 5°C, the solution formed into gel, and the solution was vacuum-dried at room temperature. | | | | | |

### 2.2.1 Hydrochloride crystal form A

XRPD results are shown in FIG. 4, and XRPD diffraction peak data are summarized in Table 13.

TGA/DSC results are shown in FIG. 5. The TGA results demonstrate that the sample has a weight loss of 0.53% when heated to 150°C. The DSC results reveal one sharp endothermic peak at 212.9°C (starting temperature).

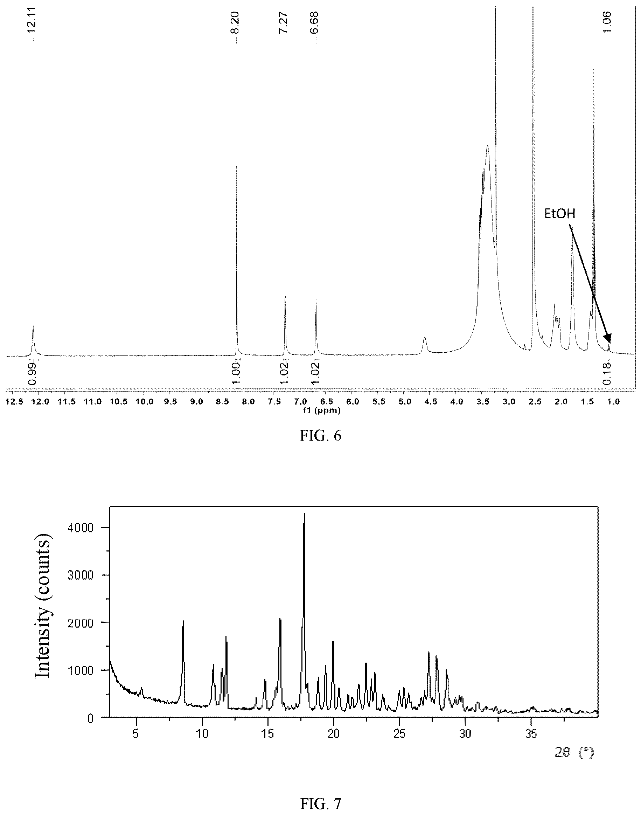

¹H NMR results are shown in FIG. 6, and a molar ratio of a residual solvent EtOH to API is 0.06 (0.7 wt%).

**Table 13 XRPD diffraction peak data of hydrochloride crystal form A**

| **20 [°]** | **Interplanar spacing [Å]** | **Relative intensity [%]** |
|---|---|---|
| 6.16 | 1395.81 | 38.56 |
| 10.87 | 1293.38 | 35.73 |
| 11.61 | 787.63 | 21.76 |
| 12.28 | 1188.99 | 32.85 |
| 14.94 | 706.85 | 19.53 |
| 16.34 | 3619.61 | 100.00 |
| 17.22 | 2219.20 | 61.31 |
| 17.69 | 248.12 | 6.85 |
| 18.14 | 140.24 | 3.87 |
| 18.44 | 446.44 | 12.33 |
| 18.87 | 1422.53 | 39.30 |
| 19.42 | 2200.20 | 60.79 |
| 20.11 | 201.27 | 5.56 |
| 21.33 | 666.67 | 18.42 |
| 21.79 | 282.42 | 7.80 |
| 22.81 | 288.01 | 7.96 |
| 23.30 | 592.38 | 16.37 |
| 23.61 | 239.10 | 6.61 |
| 24.33 | 605.33 | 16.72 |
| 24.66 | 1540.12 | 42.55 |
| 25.61 | 510.75 | 14.11 |
| 26.78 | 741.81 | 20.49 |
| 27.51 | 2887.37 | 79.77 |
| 28.44 | 299.74 | 8.28 |
| 29.55 | 434.18 | 12.00 |
| 30.27 | 181.83 | 5.02 |
| 30.79 | 127.61 | 3.53 |
| 31.35 | 213.94 | 5.91 |
| 31.73 | 177.38 | 4.90 |
| 32.69 | 120.71 | 3.33 |
| 33.01 | 305.33 | 8.44 |
| 33.81 | 134.77 | 3.72 |
| 34.68 | 175.43 | 4.85 |
| 35.06 | 241.54 | 6.67 |
| 35.77 | 124.28 | 3.43 |
| 36.71 | 56.46 | 1.56 |
| 37.35 | 66.92 | 1.85 |
| 37.85 | 54.64 | 1.51 |
| 39.39 | 115.92 | 3.20 |

### 2.2.2 Sulfate crystal form A

XRPD results are shown in FIG. 7, and XRPD diffraction peak data are summarized in Table 14.

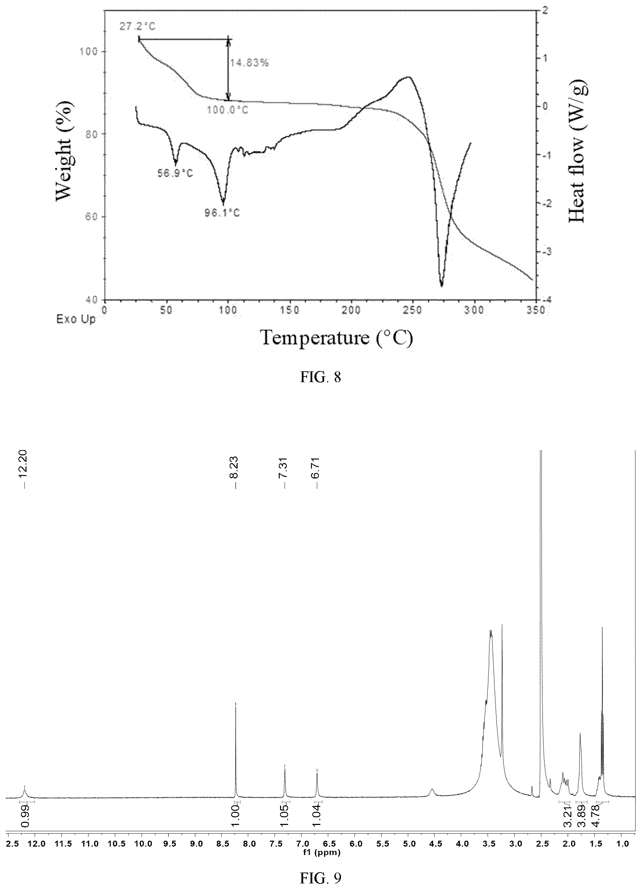

TGA/DSC results are shown in FIG. 8. The TGA results demonstrate that the sample has a stepwise weight loss of 14.83% when heated to 100°C. The DSC results reveal two endothermic signals at 56.9°C and 96.1°C (peak temperature), respectively.

1H NMR results are shown in FIG. 9, and no significant residual solvent acetone is detected.

**Table 14 XRPD diffraction peak data of Sulfate crystal form A**

| **2θ [°]** | **Interplanar spacing [Å]** | **Relative intensity [%]** |
|---|---|---|
| 5.41 | 16.34 | 4.81 |
| 8.56 | 10.33 | 43.91 |
| 10.81 | 8.18 | 22.83 |
| 11.51 | 7.69 | 21.20 |
| 11.86 | 7.46 | 37.23 |
| 14.12 | 6.27 | 5.82 |
| 14.81 | 5.98 | 15.55 |
| 15.56 | 5.69 | 9.43 |
| 15.92 | 5.57 | 49.21 |
| 17.59 | 5.04 | 39.85 |
| 17.78 | 4.99 | 100.00 |
| 18.01 | 4.93 | 13.88 |
| 18.84 | 4.71 | 17.73 |
| 19.39 | 4.58 | 23.82 |
| 19.94 | 4.45 | 35.62 |
| 20.40 | 4.35 | 11.86 |
| 21.08 | 4.21 | 8.67 |
| 21.43 | 4.15 | 7.10 |
| 21.89 | 4.06 | 14.06 |
| 22.46 | 3.96 | 25.22 |
| 22.84 | 3.89 | 15.74 |
| 23.13 | 3.84 | 19.53 |
| 23.70 | 3.75 | 8.42 |
| 24.97 | 3.57 | 10.54 |
| 25.32 | 3.52 | 12.35 |
| 25.66 | 3.47 | 9.47 |
| 26.62 | 3.35 | 7.86 |
| 26.87 | 3.32 | 11.01 |
| 27.18 | 3.28 | 31.91 |
| 27.79 | 3.21 | 28.56 |
| 28.54 | 3.13 | 21.36 |
| 29.24 | 3.05 | 7.66 |
| 29.54 | 3.02 | 9.18 |
| 29.76 | 3.00 | 8.00 |
| 30.94 | 2.89 | 5.23 |
| 32.32 | 2.77 | 2.93 |
| 35.01 | 2.56 | 2.48 |
| 36.58 | 2.46 | 1.09 |
| 37.27 | 2.41 | 2.04 |
| 37.83 | 2.38 | 2.04 |

### 2.2.3 Phosphate crystal form A

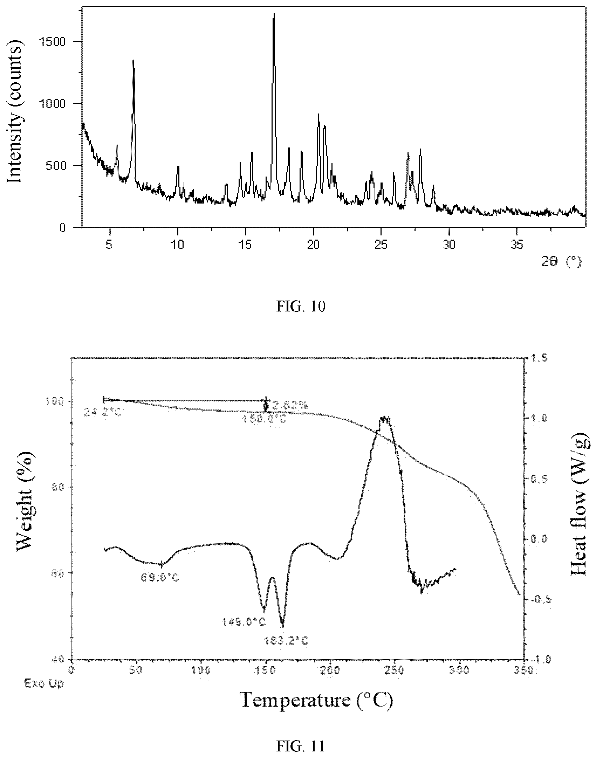

XRPD results are shown in FIG. 10, and XRPD diffraction peak data are summarized in Table 15.

TGA/DSC results are shown in FIG. 11. The TGA results demonstrate that the sample has a weight loss of 2.82% when heated to 150°C. The DSC results reveal three endothermic signals at 69.0°C, 149.0°C, and 163.2°C (peak temperature), respectively.

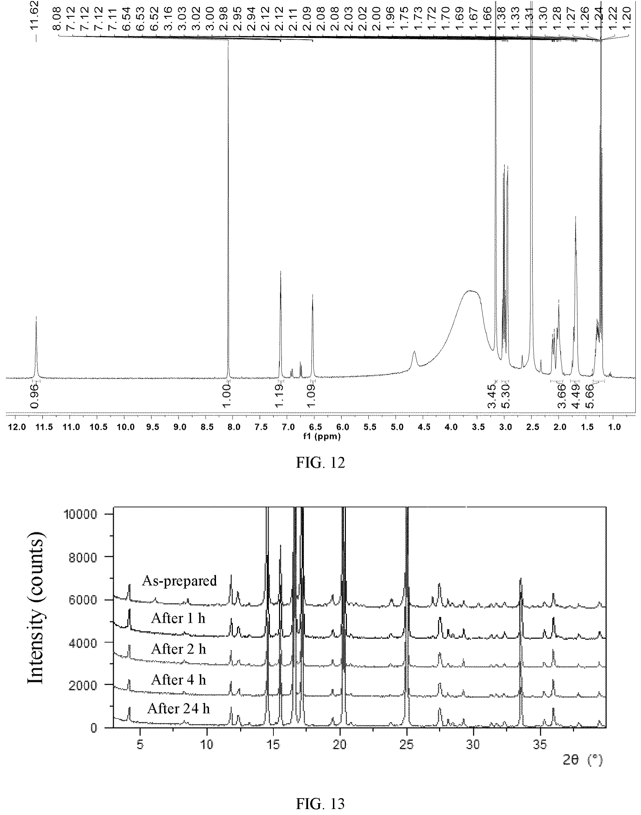

1H NMR results are shown in FIG. 12, and no significant residual solvent acetone is detected.

**Table 15 XRPD diffraction peak data of phosphate crystal form A**

| **2θ [°]** | **Interplanar spacing [Å]** | **Relative intensity [%]** |
|---|---|---|
| 5.54 | 15.95 | 12.40 |
| 6.78 | 13.03 | 64.46 |
| 10.04 | 8.81 | 16.63 |
| 10.47 | 8.45 | 7.33 |
| 13.55 | 6.53 | 9.87 |
| 14.62 | 6.06 | 17.36 |
| 15.03 | 5.89 | 10.28 |
| 15.47 | 5.73 | 27.62 |
| 16.56 | 5.35 | 12.54 |
| 17.09 | 5.19 | 100.00 |
| 18.22 | 4.87 | 30.10 |
| 19.11 | 4.64 | 28.59 |
| 20.36 | 4.36 | 48.88 |
| 20.82 | 4.27 | 41.70 |
| 21.33 | 4.17 | 21.83 |
| 23.88 | 3.73 | 12.46 |
| 24.25 | 3.67 | 17.72 |
| 25.05 | 3.55 | 12.67 |
| 25.93 | 3.44 | 18.32 |
| 26.96 | 3.31 | 27.01 |
| 27.85 | 3.20 | 31.57 |
| 28.83 | 3.10 | 12.52 |
| 30.46 | 2.93 | 2.38 |

Through comparison of the TGA/DSC patterns of the free crystal form A, the hydrochloride crystal form A, the sulfate crystal form A, and the phosphate crystal form A, it can be seen that the thermal stability of the free crystal form A and the hydrochloride crystal form A is high. The free base crystal form A has a loss on drying of 1.7% when heated to 180°C, and the hydrochloride crystal form A has a loss on drying of 0.53% when heated to 150°C. Melting points of the two are 201.5°C and 216.4°C, respectively. The sulfate crystal form A sample has a stepwise weight loss of 14.83% when heated to 100°C. The DSC results reveal two endothermic signals at 56.9°C and 96.1°C, respectively, indicating that the sulfate crystal form A is prone to decomposition or melting at lower temperature and has poor thermal stability. The phosphate crystal form A sample has a weight loss of 2.82% when heated to 150°C. The DSC results reveal three endothermic signals at 69.0°C, 149.0°C, and 163.2°C, respectively, indicating that the phosphate crystal form A has slightly poor thermal stability.

In conclusion, compared with the sulfate crystal form A and the phosphate crystal form A, the free crystal form A and the hydrochloride crystal form A not only have sharp crystal diffraction peaks, higher crystallinity, less solvent residue, a single DSC thermal signal, small TGA weight loss, and the like, but also have a higher ligand safety level. It can be seen that after the tests under more than one hundred experimental conditions in the present application, among the obtained crystal forms, only the free crystal form A and the hydrochloride crystal form A have good solid-state properties and may be developed as potential drug crystal forms.

### Example 3 Assessment of dynamic solubility

The dynamic solubility of the hydrochloride crystal form A and the free crystal form A in H₂O and biological solvents (simulated gastric fluid (SGF), fasted state simulated intestinal fluid (FaSSIF), and fed state simulated intestinal fluid (FeSSIF)) was assessed at 37°C. A specific procedure was as follows: approximately 40 mg of hydrochloride crystal form A and approximately 40 mg of free crystal form A were respectively weighed and placed into 5 mL glass vials, and 4 mL of corresponding medium (H₂O, SGF, FaSSIF and FeSSIF) was respectively added; the mixture was rotated and mixed at a rate of 25 rpm on a rotating incubator at 37°C, and sampling points were 1 h, 2 h, 4 h, and 24 h; at each sampling point, approximately 0.8 mL of suspension was taken and placed into a centrifuge tube, and centrifuged and separated (12000 rpm, 3 min, 37°C); and a supernatant was filtered with a polytetrafluoroethylene (PTFE) membrane filter (pore size of 0.45 µm), the solubility and pH of a filtrate were tested, and a solid was subjected XRPD testing (see Example 2 for the instruments and methods used).

Assessment results of dynamic solubility are summarized in Table 16, which demonstrate that the hydrochloride form A is fully soluble in H₂O, SGF, FaSSIF and FeSSIF, with a solubility of>9 mg/mL; and the solubility of the free crystal form A in SGF and FeSSIF is approximately 9 mg/mL to 10 mg/mL (the sample is not fully dissolved), and the solubility in H₂O and FaSSIF is relatively low (2.1 mg/mL to 2.4 mg/mL).

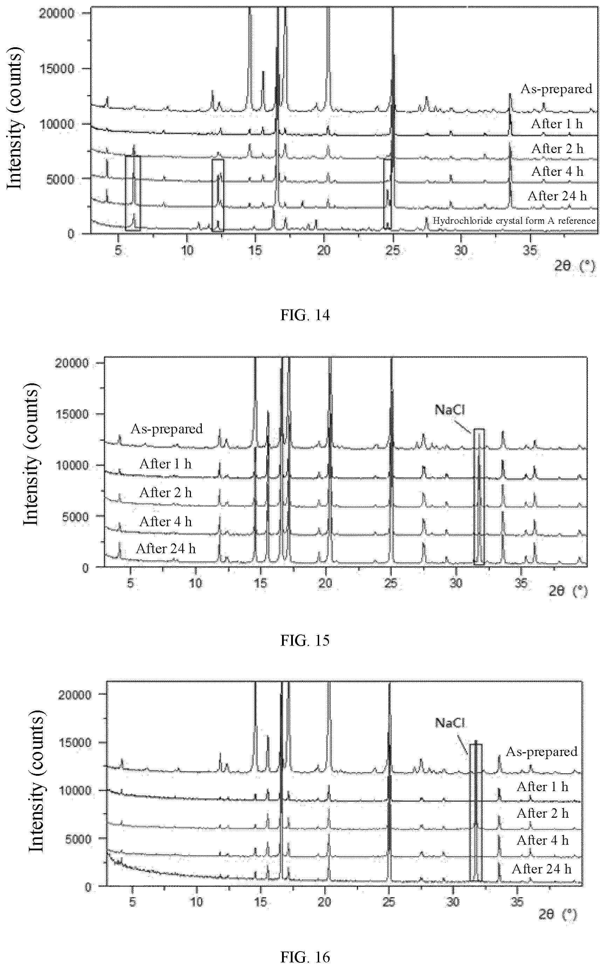

XRPD results of solid samples separated at each sampling point are shown in FIG. 13 to FIG. 16, which demonstrate that the free crystal form A does not undergo crystal transformation after 1, 2, 4, and 24 h in H₂O, FaSSIF, and FeSSIF; and a diffraction peak is observed after the hydrochloride crystal form A is dissolved in SGF for 24 h, which suggests that part of the free form reacts with Cl⁻ in the medium to form hydrochloride.

**Table 16 Assessment results of dynamic solubility**

| **Raw material** | **Medium** | **1h** | | | **2 h** | | | **4 h** | | | **24 h** | | |
|---|---|---|---|---|---|---|---|---|---|---|---|---|---|
| | | s | pH | FC | s | pH | FC | S | pH | FC | s | pH | FC |
| Hydrochlor ide crystal form A | H₂O | >9.3 | 4.0 | -- | >9.2 | 4.0 | -- | >9.5 | 4.0 | -- | >9.2 | 4.0 | -- |
| | SGF | >9.8 | 2.9 | -- | >9.9 | 3.0 | -- | >9.8 | 3.0 | -- | >10.3 | 2.9 | -- |
| | FaSSIF | >9.5 | 5.0 | -- | >9.7 | 5.1 | -- | >9.8 | 5.1 | -- | >9.6 | 5.0 | -- |
| | FeSSIF | >10.4 | 4.9 | -- | >10.2 | 4.9 | -- | >10.2 | 4.9 | -- | >10.0 | 4.8 | -- |
| Free crystal form A | H₂O | 2.1 | 6.7 | No | 2.1 | 6.6 | No | 2.2 | 6.7 | No | 2.4 | 7.3 | No |
| | SGF | 9.0 | 5.1 | No | 8.9 | 5.2 | No | 8.8 | 5.2 | No | 9.1 | 5.2 | No |
| | FaSSIF | 2.2 | 6.6 | No | 2.2 | 6.6 | No | 2.3 | 6.6 | No | 2.4 | 6.6 | No |
| | FeSSIF | 9.4 | 5.3 | No | 9.2 | 5.3 | No | 9.4 | 5.3 | No | 9.9 | 5.3 | No |

| | | | | | | | | | | | | | |
|---|---|---|---|---|---|---|---|---|---|---|---|---|---|
| S: solubility (mg/mL), calculated as free form. FC: crystal transfer. --: the sample was fully dissolved and no solid was obtained for XRPD testing. | | | | | | | | | | | | | |

### Example 4 Assessment of 24-h solubility

The 24-h solubility of the hydrochloride crystal form A and the free crystal form A in buffers (pH=2.0, 4.5, 7.4) at room temperature was assessed. A specific procedure was as follows: approximately 10 mg of hydrochloride crystal form A and approximately 10 mg of free crystal form A were respectively weighed and placed into high-performance liquid chromatography (HPLC) vials, and 1 mL of corresponding buffer was added; and the mixture was magnetically stirred at room temperature for 24 h (up to 500 rpm) A suspension was centrifuged and separated (10000 rpm, 2 min, room temperature); a supernatant was filtered with a PTFE membrane filter (pore size of 0.22 µm), the solubility and pH of a filtrate were tested, and a solid was subjected to XRPD testing (see Example 2 for the instruments and methods used).

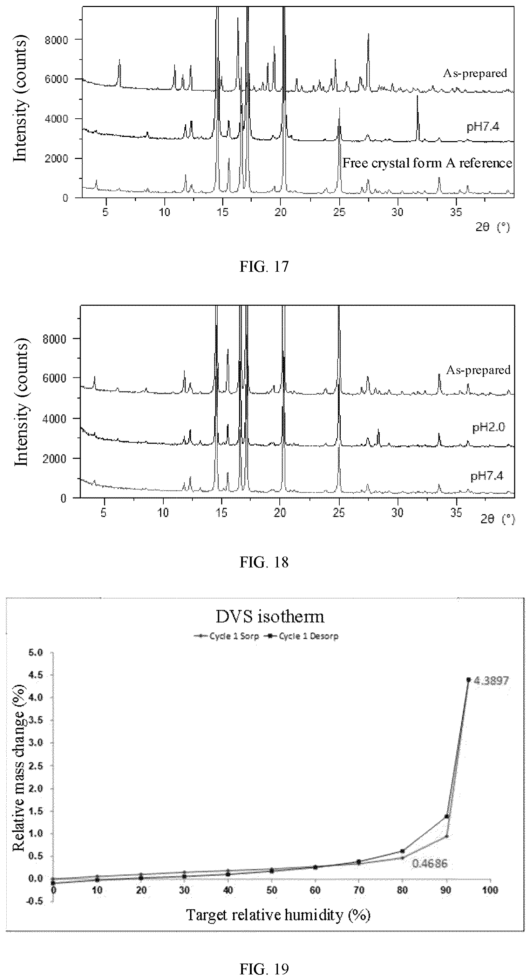

The 24-h solubility results are summarized in Table 17, and XRPD results of the separated solid samples are shown in FIG. 17 and FIG. 18. The results demonstrate that (1) the hydrochloride crystal form A has a high solubility (>9.2 mg/mL, the sample is fully dissolved) in the buffer with a pH value of 2.0 and the buffer with a pH value of 4.5; and is transformed into the free crystal form A in the buffer with a pH value of 7.4; (2) the free crystal form A has a high solubility (>9.1 mg/mL, the sample is fully dissolved) in the buffer with a pH value of 4.5; and is not fully dissolved in the buffer with a pH value of 2.0 and the buffer with a pH value of 7.4, with a solubility of 6.2 mg/mL and 1.7 mg/mL, respectively. The XRPD results demonstrate that the crystal form is not transformed after 24 h of stirring.

**Table 17 Solubility results of hydrochloride crystal form A and free crystal form A in pH buffers**

| Crystal form | Buffer (pH=2.0) | | | Buffer (pH=4.5) | | | Buffer (pH=7.4) | | |
|---|---|---|---|---|---|---|---|---|---|
| | s | pH | FC | S | pH | FC | S | pH | FC |
| Hydrochloride crystal form A | >9.5 | 3.5 | -- | >9.2 | 4.4 | -- | 2.5 | 6.2 | Yes |
| Free crystal form A | 6.2 | 5.6 | No | >9.1 | 5.0 | -- | 1.7 | 7.4 | No |

| | | | | | | | | | |
|---|---|---|---|---|---|---|---|---|---|
| S: solubility (mg/mL), calculated as free form. FC: crystal transfer. --: the sample was fully dissolved and no solid was obtained for XRPD testing. | | | | | | | | | |

### Example 5 Assessment of hygroscopicity

The hygroscopicity of the hydrochloride salt form A and the free crystal form A was assessed by DVS at 25°C under 0% RH to 95% RH. Results are summarized in Table 18 (see Example 2 for the instruments and methods used).

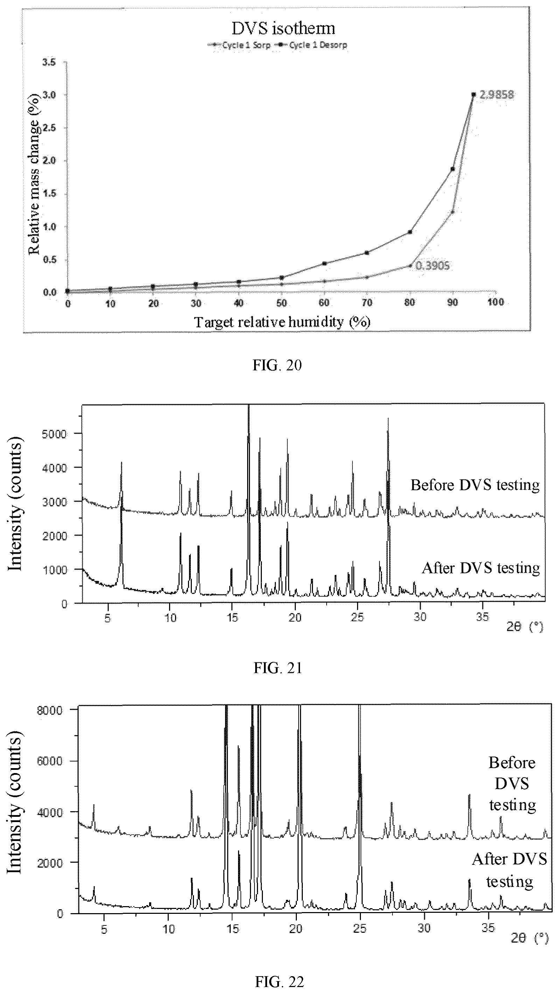

DVS results are shown in FIG. 19 and FIG. 20. Under the conditions of 25°C/80% RH, the hydrochloride crystal form A and the free crystal form A have hygroscopic weight gains of 0.47% and 0.39%, respectively, indicating slight hygroscopicity.

XRPD comparison results of the samples before and after DVS testing are shown in FIG. 21 and FIG. 22. The XRPD results demonstrate that the crystal forms of the two samples are not transformed after DVS testing.

**Table 18 Hygroscopicity results of hydrochloride crystal form A and free crystal form A**

| **Crystal form** | **Hygroscopic weight gain (wt%) at 25°C/80% RH** | **Crystal transformation after DVS** |
|---|---|---|
| Hydrochloride crystal form A | 0.47 | No |
| Free crystal form A | 0.39 | No |

### Example 6 Assessment of solid-state stability

To assess the solid-state stability of the hydrochloride form A and the free crystal form A, appropriate amounts of samples were respectively weighed, and stability experiments were set up at 80°C for 1 d in a closed state and at 25°C/60% RH and 40°C/75% RH for 4 weeks in an open state. Crystal forms of solid samples separated under different conditions were tested by XRPD to evaluate physical stability, and the purity was tested by UPLC to evaluate chemical stability (see Example 2 for the instruments and methods used). Assessment results are summarized in Table 19.

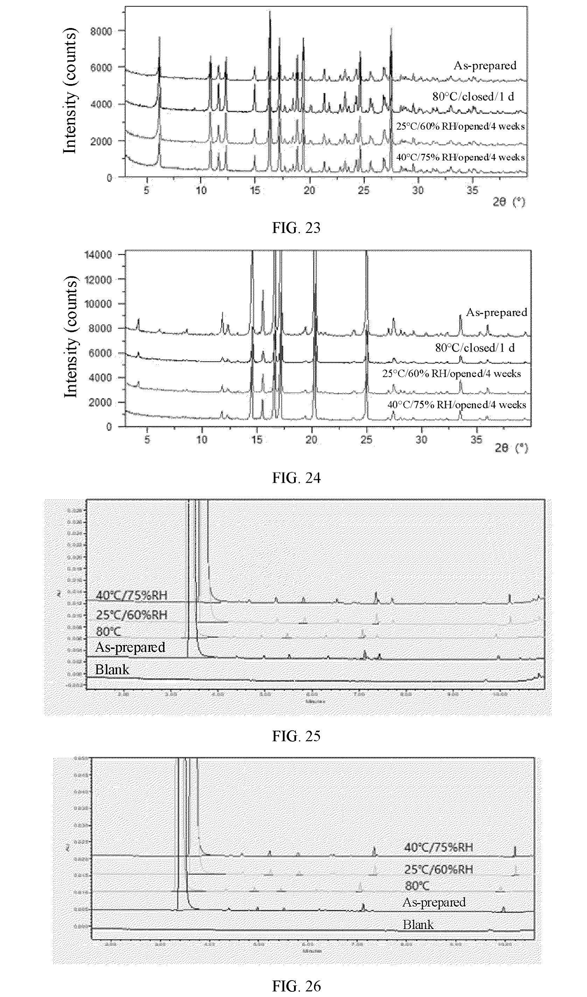

XRPD comparison results are shown in FIG. 23 and FIG. 24, UPLC results are summarized in Table 20 and Table 21, and UPLC chromatograms are shown in FIG. 25 and FIG. 26. Solid-state stability results demonstrate that the crystal forms of the hydrochloride crystal form A and the free crystal form A are not transformed under the three assessment conditions, or the purity is not reduced, indicating that the two crystal forms have high physical stability and chemical stability.

**Table 19 Solid-state stability results of hydrochloride crystal form A and free crystal form A**

| **Salt form** | **Starting purity (area%)** | **80°C/1 d** | | **25°C/60% RH/4 weeks** | | **40°C/75% RH/4 weeks** | |
|---|---|---|---|---|---|---|---|
| | | Purity (area%) | Crystal form Transform | Purity (area%) | Crystal form Transform | Purity (area%) | Crystal form Transform |
| Hydrochloride crystal form A | 99.82 | 99.87 | No | 99.88 | No | 99.88 | No |
| Free crystal form A | 99.72 | 99.71 | No | 99.71 | No | 99.72 | No |

| | | | | | | | |
|---|---|---|---|---|---|---|---|
| **[00255]** *: the crystallinity was reduced. | | | | | | | |

**Table 20 UPLC results for stability assessment of hydrochloride crystal form A**

| **#Peak** | **RRT** | **Area (%)** | | | |
|---|---|---|---|---|---|
| | | Starting | 80°C/1 d | 25°C/60% RH/4 weeks | 40°C/75% RH/4 weeks |
| 1 | 1.00 | 99.82 | 99.87 | 99.88 | 99.88 |
| 2 | 1.62 | 0.05 | 0.05 | 0.05 | 0.05 |
| 3 | 2.10 | 0.08 | 0.08 | 0.07 | 0.07 |
| 4 | 2.19 | 0.06 | <0.05 | <0.05 | <0.05 |

| **[00257]** Table 21 UPLC results for stability assessment of free crystal form A | | | | | |
|---|---|---|---|---|---|
| **#Peak** | **RRT** | **Area (%)** | | | |
| | | Starting | 80°C/1 d | 25°C/60% RH/4 weeks | 40°C/75% RH/4 weeks |
| 1 | 1.00 | 99.72 | 99.71 | 99.71 | 99.72 |
| 2 | 1.47 | 0.05 | 0.06 | 0.06 | 0.06 |
| 3 | 1.63 | 0.05 | 0.05 | 0.05 | 0.05 |
| 4 | 2.10 | 0.10 | 0.10 | 0.10 | 0.10 |
| 5 | 2.94 | 0.07 | 0.08 | 0.07 | 0.07 |

### Example 7 Assessment of powder properties

Powder properties of the hydrochloride crystal form A and the free crystal form A were assessed, including flowability and morphology, to understand the basic powder properties of each salt form and the free crystal form A.

The flowability was assessed by testing the bulk density/tapped density and angle of repose of the samples (see Example 2 for the instruments and methods used). A method for testing the density and the tapped density included: (1) a certain mass of to-be-assessed sample was placed into a 5 mL measuring cylinder, and the volume at this moment was recorded. The bulk density was calculated by dividing the mass of the sample by the volume at this moment. (2) The measuring cylinder was slightly tapped 200 times, the final volume was recorded, and the tapped density was calculated by dividing the mass of the sample by the final volume. (3) Three parallel tests were conducted. A method for testing the angle of repose included: (1) a funnel was fixed vertically to a bottom surface, and the material was slowly placed into the funnel. (2) A well-proportioned cone of the material was formed on the bottom surface. The height and bottom surface diameter of the cone were measured. (3) Three parallel tests were conducted.

Results of the bulk density/tapped density are summarized in Table 22, which demonstrate that the bulk density and the tapped density of the hydrochloride crystal form A are 0.14 g/cm³ and 0.24 g/cm³, respectively, and a calculated Carr index is 41%; and the bulk density and the tapped density of the free crystal form A are 0.11 g/cm³ and 0.23 g/cm³, respectively, and a calculated Carr index is 51%.

The results of the angle of repose are summarized in Table 23, which demonstrate that the angels of repose of the powder samples of the hydrochloride crystal form A and the free crystal form A are 33.9° and 47.1°, respectively. In conclusion, the samples of the hydrochloride crystal form A and the free crystal form A have small Carr indices and angles of repose, and thus have high flowability.

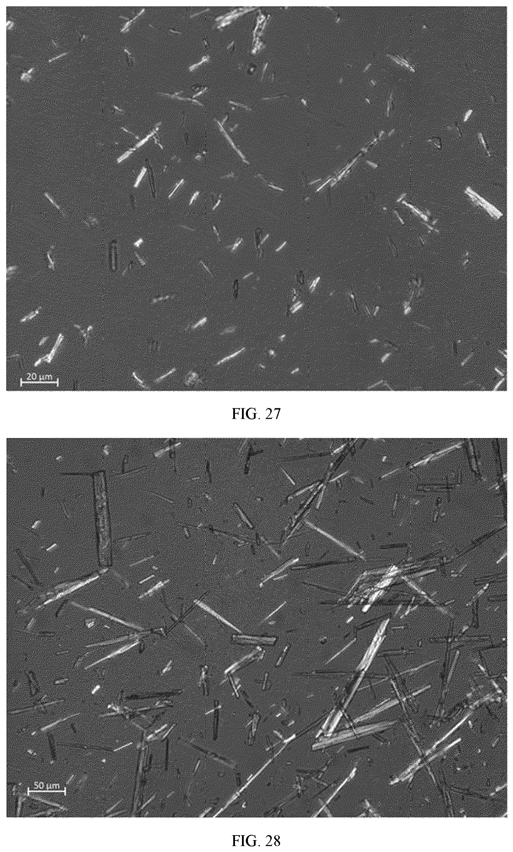

PLM results are shown in FIG. 27 and Fig. 28. Under a polarizing microscope, it is observed that the hydrochloride crystal form A is a rob-shaped crystal; and the free crystal form A is a needle-shaped or long sheet-like crystal.

**Table 22 Test results of bulk density/ tapped density**

| **Crystal form** | **Number of tests** | **Bulk density (g/cm³)** | **Average bulk density (g/cm³)** | **Tapped density (g/cm³)** | **Average tapped density (g/cm³)** | **Carr index** |
|---|---|---|---|---|---|---|
| Hydrochloride crystal form A | 1 | 0.14 | 0.14 | 0.23 | 0.24 | 41% |
| | 2 | 0.14 | | 0.25 | | |
| | 3 | 0.13 | | 0.23 | | |
| Free crystal form A | 1 | 0.11 | 0.11 | 0.23 | 0.23 | 51% |
| | 2 | 0.11 | | 0.23 | | |
| | 3 | 0.11 | | 0.22 | | |

| | | | | | | |
|---|---|---|---|---|---|---|
| Carr index = (tapped density - bulk density) / tapped density | | | | | | |

**Table 23 Test results of angles of repose**

| **Crystal form** | **Number of tests** | **Cone height (cm)** | **Cone diameter (cm)** | **Angle of repose (°)** | **Average angle of repose (°)** |
|---|---|---|---|---|---|
| Hydrochloride crystal form A | 1 | 1.6 | 4.8 | 33.7 | 33.9 |
| | 2 | 1.9 | 5 | 37.2 | |
| | 3 | 1.4 | 4.7 | 30.8 | |
| Free crystal form A | 1 | 2.7 | 4.7 | 49.0 | 47.1 |
| | 2 | 2.6 | 4.8 | 47.3 | |
| | 3 | 2.5 | 5.0 | 45.0 | |

Angle of repose *α* = tan⁻¹ (h/D), where h is the cone height, and D is the cone diameter.

### Example 8 Assessment of mechanical stability

The hydrochloride crystal form A and the free crystal form A were taken as starting samples, and ground manually and compressed by a tablet press (350 MPa pressure), respectively, the ground and compressed samples were subjected to XRPD testing to assess mechanical stability of the samples (see Example 2 for the instruments and methods used). Specific results are summarized in Table 24.

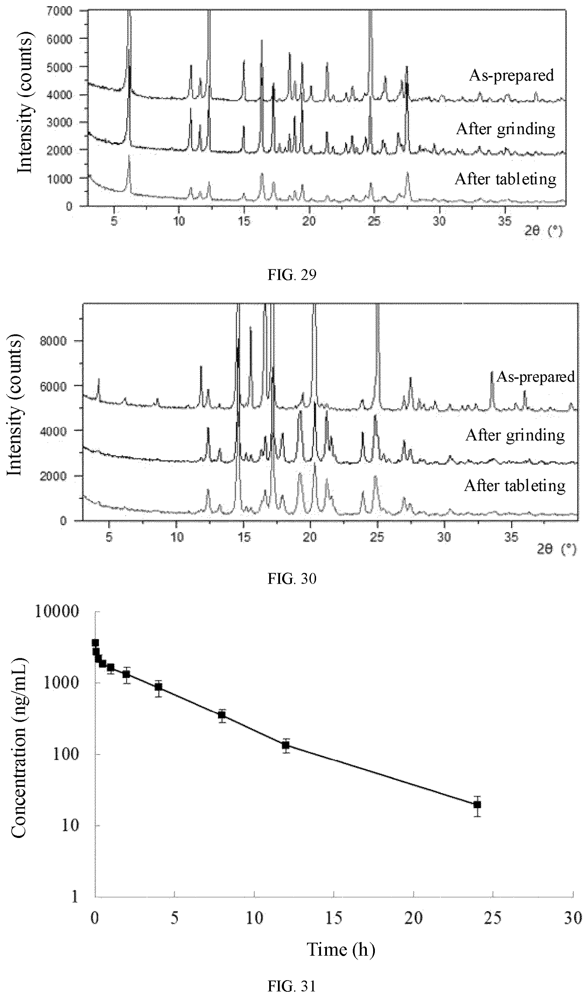

XRPD results before and after grinding and tableting are shown in FIG. 29 and FIG. 30, which demonstrate that: (1) after grinding, the crystal form and crystallinity of the hydrochloride crystal form A are remained unchanged, the crystal form of the free crystal form A remains unchanged but some diffraction peaks are broadened, which suggests that the crystallinity may be slightly reduced; and (2) after tableting, the crystal forms of the hydrochloride crystal form A and the free crystal form A remain unchanged, but the crystallinity is slightly reduced (some diffraction peaks are broadened, and some weak diffraction peaks disappear).

**Table 24 Assessment results of mechanical stability**

| **Crystal form** | **Results of mechanical stability** | |
|---|---|---|
| | After grinding | After tableting |
| Hydrochloride crystal form A | The crystal form remains unchanged | The crystal form remains unchanged, but the crystallinity is slightly reduced |
| Free crystal form A | The crystal form remains unchanged, but the crystallinity is slightly reduced | The crystal form remains unchanged, but the crystallinity is slightly reduced |

In conclusion, the hydrochloride crystal form A and the free crystal form A have high solid-state stability and flowability, low hygroscopicity, high solubility, and excellent comprehensive physical and chemical properties, and thus may be developed as potential drug crystal forms and have good application prospects.

### Example 9 Study on pharmacokinetics of free crystal form A

### 1. Experimental objective

This experiment was designed to study the pharmacokinetic properties of the free crystal form A (prepared in Example 1, sometimes referred to as a drug in this example) in male and female Beagle dogs, including 1) a study of a single intravenous injection (i.v.) at 2 mg/kg; 2) a dose escalation study of intragastric (i.g.) administration at 2 mg/kg, 6 mg/kg, and 20 mg/kg; and 3) a study of 7 consecutive days of intragastric administration at a dose of 6 mg/kg.

### 2. Experimental animals

Ordinary Beagle dogs used in this experiment were purchased from Beijing Marshall Bioresources Co., Ltd., with the license No. SCXK (Jing) 2016-0001, the animal qualification certificate No. 1103182011000078 and 1103182011000079, and the experimental animal use permit No. SYXK (Su) 2021-0045. The animal room was well ventilated and equipped with air conditioners. The temperature was maintained at 16°C to 26°C and the humidity was maintained at 40% to 70%. Artificial lighting was adopted, with 12 h of light and 12 h of dark. At the time of the first administration, a body weight range of the male Beagle dogs was 8.0 kg to 11.6 kg, and a body weight range of the female Beagle dogs was 6.9 kg to 11.3 kg.

### 3. Preparations

### (1) Intravenous injection

A solvent for an intravenous injection group was DMA: 30% Solutol-HS 15: Saline = 10: 10: 80 (v/v/v).

150.34 mg of free crystal form A was weighed and placed into a glass vial, 7.517 mL of DMA was added, the mixture was subjected to vortex mixing and sonication until particles were dissolved, 7.517 mL of 30% (w/v) Solutol-HS aqueous solution with vortex mixing, 60.136 mL of Saline was added with vortex mixing to obtain a colorless solution at a final concentration of 2 mg/mL, and the solution was filtered with a 0.45 µm nylon membrane filter (PALL) and administered to animals in a group A.

### (2) Oral gavage

A solvent for intragastric administration groups was a 0.5% carboxymethyl cellulose sodium (CMC-Na) aqueous solution.

150.43 mg of free crystal form A was weighed and placed into a glass vial, 376.075 mL of 0.5% CMC-Na aqueous solution was added, and the mixture was subjected to vortex mixing and sonication until particles were dissolved, to obtain a colorless solution at a final concentration of 0.4 mg/mL, which was administered to animals in a group B.

450.58 mg of free crystal form A was weighed and placed into a glass vial, 375.483 mL of 0.5% CMC-Na aqueous solution was added, and the mixture was subjected to vortex mixing and sonication until particles were dispersed, to obtain a white suspension at a final concentration of 1.2 mg/mL, which was administered to animals in a group C on the first day. Preparations were administered to the group C on the second day to the seventh day were prepared in the same way as that on the first day.

1500.94 mg of free crystal form A was weighed and placed into a glass vial, 375.235 mL of 0.5% CMC-Na aqueous solution was added, and the mixture was subjected to vortex mixing and sonication until particles were dispersed, to obtain a white suspension at a final concentration of 4 mg/mL, which was administered to animals in a group D.

All the preparations were freshly prepared on the day of administration, and sampled for determination of preparation concentration. Only the preparations administered to the group D on the first day, the fourth day, and the seventh day were sampled for determination of preparation concentration.

### 4. Experimental groups

All the animals in this experiment were randomly divided into 4 groups (3 dogs/sex/group). Grouping and administration information is summarized in Table 25. The animals in the group A, the group B, and the group D were fasted before administration, and the animals in the group C were fasted before the first administration and the last administration. All the animals were fasted for at least 12 h before administration, and food was resumed 4 h after administration. All the animals had free access to water and food during the experiment.

**Table 25 Animal groups and administration methods**

| **Group** | **Number of animals** | | **Dose (mg/kg)** | **Volume (mL/kg)** | **Concentration (mg/mL)** | **Administration route** | **Number of administrations** |
|---|---|---|---|---|---|---|---|
| | **Male** | **Female** | | | | | |
| A | 3 | 3 | 2 | 1 | 2 | Intravenous injection | Single |
| B | 3 | 3 | 2 | 5 | 0.4 | Intragastric | Single |
| C | 3 | 3 | 6 | 5 | 1.2 | Intragastric | 7 times (once a day) |
| D | 3 | 3 | 20 | 5 | 4 | Intragastric | Single |

### 5. Sample collection and treatment

The animals in the group A were intravenously injected with the drug at 2 mg/kg, and blood samples were collected from the jugular vein before and 0.033 h, 0.083 h, 0.25 h, 0.5 h, 1 h, 2 h, 4 h, 8 h, 12 h, and 24 h after administration. The animals in the group B and the group D received single intragastric administration of the drugs at 2 mg/kg and 20 mg/kg, respectively, and blood samples were collected from the jugular vein before and 0.083 h, 0.25 h, 0.5 h, 1 h, 2 h, 4 h, 8 h, 12 h, and 24 h after administration. The animals in group C received intragastric administration of the drug at 6 mg/kg once a day for seven consecutive days. Blood samples were collected from the jugular vein before the first administration and the seventh administration and 0.083 h, 0.25 h, 0.5 h, 1 h, 2 h, 4 h, 8 h, 12 h, and 24 h after administration. Blood samples were collected from the jugular vein before administration and 0.5 h after administration from the second administration to the sixth administration. The sample collected before the second administration was the sample collected 24 h after the first administration.

The blood samples from the animals were collected through the jugular vein, with a blood sample volume of 0.5 mL/time. All collected whole blood samples were placed in centrifuge tubes containing ethylene diamine tetraacetic acid potassium salt (EDTA-K2), and the centrifuge tubes were turned upside down to allow the anticoagulant and blood to be fully mixed. All the samples were placed on wet ice before centrifugation and centrifuged at 1524 g for 10 min to separate plasma. The plasma samples were transferred to new centrifuge tubes and stored at -90°C to 60°C until analysis.

The drug concentration in the plasma of each Beagle dog was determined by liquid chromatography with tandem mass spectrometry (LC-MS/MS). The obtained plasma concentration data was analyzed by using the pharmacokinetic processing software WinNonlin 8.0 with a non-compartmental model to calculate relevant pharmacokinetic parameters.

### 6. Experimental results

The animals were clinically observed before administration, before and after each blood sampling time point, and after administration, and no obvious abnormalities were found. The plasma concentration-time curves of the groups are shown in FIG. 31 to FIG. 33, and main pharmacokinetic parameters are summarized in Table 26 and Table 27.

**Table 26 Main pharmacokinetic parameters of Beagle dogs intravenously injected with drug at 2 mg/kg**

| Animal | C₀ (ng/mL) | t_{1/2} (h) | MRT₀₋ₜ (h) | AUC₀₋ₜ (h*ng/mL) | | CL (mL/kg/min) | V_{dSS} (L/kg) |
|---|---|---|---|---|---|---|---|
| Total weight | Mean | 4250 | 3.68 | 4.30 | 9940 | 3.40 | 0.939 |
| | SD | 743 | 0.426 | 0.366 | 1850 | 0.578 | 0.214 |

After the Beagle dogs are intravenously injected with the drug at 2 mg/kg, male-to-female ratios of C₀ and AUC₀₋ₜ of the drug are 0.758 and 0.748, respectively (within the range of 0.5 times to 2 times). There is no significant gender difference in systemic exposure. The total clearance (CL) of the drug in the male and female Beagle dogs was equivalent to 0.110 times the liver blood flow (approximately 31 mL/min/kg, Davies and Morris (1993)) of the Beagles dog, indicating that the drug is eliminated slowly in the Beagle dog. The steady-state volume of distribution (V_{dss}) is 1.57 times the total body fluid volume (approximately 0.60 L/kg, Davies and Morris (1993)) of the Beagle dog, indicating that it tends to be distributed in tissues.

**Table 27 Main pharmacokinetic parameters of Beagle dogs receiving intragastric administration of drug at different doses**

| Dose (mg/kg) | Animal | | t_{1/2} (h) | Tₘₐₓ (h) | Cₘₐₓ (ng/mL) | AUC₀₋ₜ (h*ng/mL) | MRT₀₋ₜ (h) | F% |
|---|---|---|---|---|---|---|---|---|
| 2 | Male/female | Mean | 3.89 | 1.33 | 1350 | 8750 | 5.37 | 88.0 |
| | | SD | 0.586 | 1.33 | 243 | 1160 | 0.904 | 11.6 |
| 6 First administration | Male/female | Mean | 4.20 | 0.958 | 4710 | 30000 | 5.49 | 100.0 |
| | | SD | 0.514 | 0.813 | 1370 | 7780 | 0.512 | 26.1 |
| 20 | Male/female | Mean | 4.17 | 0.792 | 13300 | 101000 | 5.20 | 101.0 |
| | | SD | 0.247 | 0.332 | 1480 | 11800 | 0.130 | 11.9 |
| 6 Seventh administration | Male/female | Mean | 4.10 | 0.750 | 4540 | 26600 | 5.12 | NA |
| | | SD | 0.379 | 0.612 | 1350 | 4760 | 0.463 | NA |

After the Beagle dogs receive single intragastric administration of the drugs at 2 mg/kg, 6 mg/kg, and 20 mg/kg, respectively, the average time to peak drug concentration ranges from 0.792 h to 1.33 h after administration. After dose correction, the oral bioavailability F% calculated by the average AUC₀₋ₜ is 88.0%, 100.0%, and 101.0%, respectively.

After the Beagle dogs receive single intragastric administration of the drug at 2 mg/kg, 6 mg/kg, and 20 mg/kg, respectively, male-to-female ratios of Cₘₐₓ of the drug at the three doses are 1.36, 0.810, and 0.894, respectively, and male-to-female ratios of AUC₀₋ₜ are 1.10, 0.716, and 0.877, respectively. There is no significant gender difference in systemic exposure (the ratios are all within the range of 0.5 times to 2 times).

After the male and female Beagle dogs receive intragastric administration of the drug at 2 mg/kg, 6 mg/kg, and 20 mg/kg, respectively, the unit dose Cₘₐₓ values of the unit dose are 675 kg*ng/ml/mg, 785 kg*ng/ml/mg, and 665 kg*ng/ml/mg, respectively, and AUC₀₋ₜ values of the unit dose are 4380 ng*h*kg/mL/mg, 4990 ng*h*kg/mL/mg, and 5030 ng*h*kg/mL/mg, respectively. The results indicate that within the dose range of 2 mg/kg to 20 mg/kg, the systemic exposure of the drug in the Beagle dogs exhibits a dose-related linear increase (the unit dose Cₘₐₓ ratio and unit dose AUC₀₋ₜ ratio of 20 mg/kg and 2 mg/kg are 0.985 and 1.15, respectively, both within the range of 0.5 times to 2 times).

After the beagle dogs receive intragastric administration of the drug at 6 mg/kg for 7 consecutive days, male-to-female ratios of Cₘₐₓ and AUC₀₋ₜ of the seventh administration and the first administration are 0.964 and 0.887, respectively. No significant drug accumulation is observed.

In conclusion, the Beagle dogs do not exhibit a significant gender difference in systemic exposure after receiving intragastric administration of the drug at 2 mg/kg, 6 mg/kg, and 20 mg/kg, respectively. Within the dose range of 2 mg/kg to 20 mg/kg, the systemic exposure in the experimental animals exhibits a dose-related linear increase, and there is no significant drug accumulation after 7 consecutive days of administration.

### Example 10 Study on pharmacokinetics of hydrochloride crystal form A

### 1. Experimental objective

This experiment was designed to study the pharmacokinetic properties of the hydrochloride crystal form A (prepared in Example 2, sometimes referred to as a drug in this example) in male and female Beagle dogs.

### 2. Experimental animals

Three 31-month-old male Beagle dogs used in this experiment were purchased from Beijing Marshall Bioresources Co., Ltd., with the animal qualification certificate No. 110318201100056783.

### 3. Preparations

A solvent for intragastric administration groups was a 0.5% CMS-Na aqueous solution.

260.20 mg of hydrochloride crystal form A (prepared in Example 2) was weighed and placed in a glass vial, 193.827 mL of 0.5% CMC-Na aqueous solution, and the mixture was subjected to vortex mixing and sonication until particles were dissolved, to obtain a colorless solution at a final concentration of 1.2 mg/mL, which was administered to animals in a group A.

### 4. Experimental groups

In this experiment, three male Beagle dogs were randomly selected for administration. Administration methods are summarized in Table 28. All the animals were fasted for at least 12 h before administration and were fed again 4 h after administration. All the animals had free access to water during the experiment.

**Table 28 Administration methods for animals**

| **Group** | **Number of animals** | **Dose (mg/kg)** | **Volume (mL/kg)** | **Concentration (mg/mL)** | **Administration route** | **Number of administrations** |
|---|---|---|---|---|---|---|
| A | 3 | 6 | 5 | 1.2 | Intragastric | Single |

### 5. Sample collection and treatment

The animals in the group A received single intragastric administration of the drug at 6 mg/kg, and blood samples were collected from the jugular vein before administration and 0.083 h, 0.25 h, 0.5 h, 1 h, 2 h, 4 h, 8 h, 12 h, and 24 h after administration.

The blood samples from the animals were collected through the jugular vein, with a blood sample volume of 0.5 mL/time. All collected whole blood samples were placed in centrifuge tubes containing EDTA-K2, and the centrifuge tubes were turned upside down to allow the anticoagulant and blood to be fully mixed. All the blood samples were placed on wet ice before centrifugation and centrifuged at 1500 g for 10 min to separate plasma. The plasma samples were transferred to sample tubes and stored at -40°C to 20°C until analysis.

The drug concentration in the plasma of each Beagle dog was detected by an LC-MS/MS method. The LC-MS/MS detection method was as follows:
Instrument: LC-MS/MS (Triple Quad 5500+: LC-MS-MS-023).
MS conditions: electrospray ionization (ESI) positive ion mode; multiple reaction monitoring (MRM); m/z 336.2/149.1.
Chromatographic column: Waters Xselect HSS T3, 3.5 µm, 2.1×50 mm.
Mobile phase A: H₂O (0.1% formic acid + 5mM NH₄OAc).
Mobile phase B: MeOH: ACN=1: 1 (0.1% formic acid).
Flow rate: 0.50 mL/min.
Injection volume: 5 µL.
Retention time: 1.24 min.

An elution program is shown in Table 29.

**Table 29 Elution program**

| Time (min) | Mobile phase B (%) |
|---|---|
| 0.50 | 16 |
| 1.50 | 35 |
| 1.60 | 95 |
| 2.20 | 95 |
| 2.21 | 16 |
| 3.00 | Stop |

### 6. Experimental results

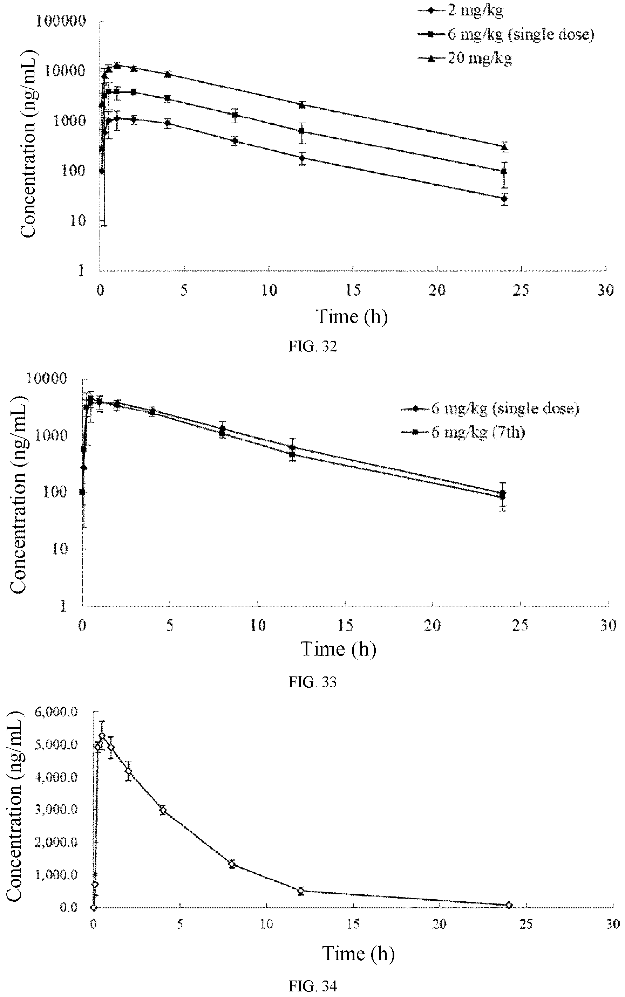

The animals were clinically observed before administration, before and after each blood sampling time point, and after administration, and no obvious abnormalities were found. Plasma concentration-time curves are shown in FIG. 34, and main pharmacokinetic parameters are summarized in Table 30.

**Table 30 Pharmacokinetic parameters of Beagle dog receiving single intragastric administration of drug at 6 mg/kg**

| PK parameter | Mean | SD | CV (%) |
|---|---|---|---|
| Kₑₗ (1/h) | 0.194 | 0.0167 | 8.6 |
| T_{1/2} (h) | 3.59 | 0.295 | 8.2 |
| Tₘₐₓ (h) | 0.417 | 0.144 | 34.6 |
| Cₘₐₓ (ng/mL) | 5290 | 405 | 7.7 |
| AUC₀₋ₜ (ng·h/mL) | 31700 | 208 | 0.7 |
| AUC_{0-inf}(ng·h/mL) | 32100 | 361 | 1.1 |
| MRT₀₋ₜ(h) | 4.84 | 0.350 | 7.2 |
| MRT_{0-inf}(h) | 5.11 | 0.374 | 7.3 |

As shown in the table, after the Beagle dog receives single intragastric administration of the drug at 6 mg/kg, Tₘₐₓ is 0.417 h, indicating that the hydrochloride crystal form A of the present application exhibits shorter time to peak drug concentration. For the marketed drug Apoquel^{®} (with oclacitinib as the active ingredient), after an experimental dog receives the drug orally, Tₘₐₓ is less than 1 h (P7, CVMP assessment report for APOQUEL (EMEA/V/C/002688/0000), EMA/481054/2013). It can be seen that compared with the marketed drug Apoquel^{®}, the hydrochloride crystal form A of the present application has better or at least equivalent advantages in terms of the time to peak drug concentration in vivo.

### Example 11 Acute toxicity test of free crystal form A

### 1. Experimental objective

After an experimental animal received single oral administration of the free crystal form A (prepared in Example 1) of the test substance, toxicity reactions produced by the experimental animal were observed within a short period of time to obtain a preliminary understanding of toxicity characteristics and dose-response relationship of the test substance.

### 2. Experimental animals

Sprague-Dawely rats (SPF grade) used in this experiment were purchased from Zhejiang Charles River Experimental Animal Technology Co., Ltd. with the production license No. SCXK (Zhe) 2019-0001 and the animal qualification certificate No. 20220107Aazz0619000738 and 20220107Aazz0619000691. A body weight range of males was 206.6 g to 234.5 g, and a body weight range of females was 188.4 g to 205.9 g.

### 3. Preparations

A solvent for intragastric administration was a 0.5% CMC-Na aqueous solution. 17.5031 g of CMC-Na (800-1200 mPa.s) was weighed and placed into a suitable container, 3500 mL of deionized water was added, the mixture was stirred until uniform to obtain a colorless clear liquid, and the liquid was stored at room temperature until use.

100 mg/mL free crystal form A solution: 9000.4 mg of free crystal form A was weighed and placed into a wide-mouth bottle with a line of 90 mL, an appropriate amount of 0.5% CMC-Na (800-1200 mPa.s) aqueous solution was added to the foregoing container, the mixture was ultrasonically stirred until uniform, an appropriate amount of 0.5% CMC-Na (800-1200 mPa.s) aqueous solution was added to make up to 90 mL, and the mixture was ultrasonically stirred until uniform. Sampling was performed as required to obtain a milky white suspension.

55.9 mg/mL free crystal form A solution: 44.7 mL of 100 mg/mL free crystal form A solution was taken and placed into a suitable container, 35.3 mL of 0.5% CMC-Na (800-1200 mPa.s) aqueous solution was added to the foregoing container, and the mixture was subjected to vortex mixing until uniform. Sampling was performed as required to obtain a milky white suspension.

31.2 mg/mL free crystal form A solution: 44.7 mL of 55.9 mg/mL free crystal form A solution was taken and placed into a suitable container, 35.3 mL of 0.5% CMC-Na (800-1200 mPa.s) aqueous solution was added to the foregoing container, and the mixture was subjected to vortex mixing until uniform. Sampling was performed as required to obtain a milky white suspension.

17.4 mg/mL free crystal form A solution: 44.7 mL of 31.2 mg/mL free crystal form A solution was taken and placed into a suitable container, 35.3 mL of 0.5% CMC-Na (800-1200 mPa.s) aqueous solution was added to the foregoing container, and the mixture was subjected to vortex mixing until uniform. Sampling was performed as required to obtain a milky white suspension.

9.7 mg/mL free crystal form A solution: 44.7 mL of 17.4 mg/mL free crystal form A solution was taken and placed into a suitable container, 35.3 mL of 0.5% CMC-Na (800-1200 mPa.s) aqueous solution was added to the foregoing container, and the mixture was subjected to vortex mixing until uniform. Samples were taken as required to obtain a white suspension.

### 4. Experimental groups

In this experiment, all animals were randomly divided into 5 groups (5 animals/sex/group), received the drug at 97 mg/kg, 174 mg/kg, 312 mg/kg, 559 mg/kg, and 1000 mg/kg, respectively, and received signal intragastric administration orally. The animals were observed for 7 d. Grouping and administration information are summarized in Table 31. All animals received administration according to their latest weighed body weight. The drug was stirred for at least 10 min before administration, and continuously stirred during administration until the administration of the sample at this concentration was completed. The animals were fasted for 11 h to 12 h before administration and resumed eating approximately 2 h after administration, with no restriction on drinking water.

All animals underwent a comprehensive physical examination by a veterinarian before administration. During the acclimation period, cage-side observation was performed once or twice a day, and detailed clinical observation was performed once a day. During the experiment, cage-side observation was performed twice a day (once on the day of dissection), and detailed clinical observation was performed once or twice a day. All surviving animals were weighed once on the day of random grouping (Day-1), Day 1 (before administration), Day 3, Day 5, Day 7, and Day 8; and their 24±1-hour food consumption was measured once on Day 1 (Day 1 to Day 2), Day 2 (Day 2 to Day 3), and Day 6 (Day 6 to Day 7). At the end of the experimental (Day 8), all surviving animals were euthanized by carbon dioxide inhalation and abdominal aorta/vein bleeding and then subjected to gross anatomical examination. Animals that died during the experiment were also subjected to gross anatomical examination.

**Table 31 Animal groups and administration methods**

| **Group** | **Number of animals** | | **Dose (mg/kg)** | **Volume (mL/kg)** | **Concentration (mg/mL)** | **Administration route** | **Number of administrations** |
|---|---|---|---|---|---|---|---|
| | **Male** | **Female** | | | | | |
| A | 5 | 5 | 97 | 10 | 9.7 | Intragastric | Single |
| B | 5 | 5 | 174 | 10 | 17.4 | Intragastric | Single |
| C | 5 | 5 | 312 | 10 | 31.2 | Intragastric | Single |
| D | 5 | 5 | 559 | 10 | 55.9 | Intragastric | Single |
| E | 5 | 5 | 1000 | 10 | 100 | Intragastric | Single |

### 5. Experimental results

Average drug recovery rates of the test sample preparations at various concentrations were within a range of 105.8% to 109.8%, and the %RSD of the upper, middle, and lower layers of the low and high concentration samples is ≤ 0.89, which met the acceptance criteria of 100±15% average recovery at theoretical concentration with %RSD ≤ 10.

Under the conditions of this experiment, after the SD rats receive single intragastric administration orally, the LD50 of the male rats is 592 mg/kg, with a 95% confidence interval of 429 mg/kg to 818 mg/kg, and a maximum tolerance dose (MTD) of 312 mg/kg; and the LD50 of the female rats is 418 mg/kg, with a 95% confidence interval of 313 mg/kg to 558 mg/kg, and a maximum tolerance dose of 174 mg/kg. The LD50 of the marketed drug Apoquel^{®} for rats is 310 mg/kg (P9, CVMP assessment report for APOQUEL (EMEA/V/C/002688/0000), EMA/481054/2013), indicating that the free crystal form A of the present application has lower toxicity.

### Example 12 Study on urine and fecal excretion of Beagle dogs receiving single intragastric administration of free crystal form A

### 1. Experimental objective

This experiment was designed to study an excretion process of the free crystalline form A (prepared in Example 1) in the feces and urine of male and female Beagle dogs after single intragastric administration.

### 2. Experimental animals

Ordinary Beagle dogs used in this experiment were purchased from Beijing Marshall Bioresources Co., Ltd., with the production license No. SCXK (Jing) 2016-0001 and the qualification certificate No. 1103182011000078 (male) and 1103182011000079 (female). The experimental animals were kept in an animal room of Suzhou 3D BioOptima Co., Ltd., with the license No. SYXK (Su) 2021-0045. The animal room was well ventilated and equipped with air conditioners. The temperature was maintained at 16°C to 26°C and the humidity was maintained at 40% to 70%. Artificial lighting was adopted with 12 h of light and 12 h of dark. A body weight range of the male Beagle dogs at the moment of administration on the day of the experiment was 8.6 kg to 10.5 kg, and a body weight range of the female Beagle dogs was 7.6 kg to 9.0 kg.

### 3. Preparations

A solvent for intragastric administration was a 0.5% CMC-Na aqueous solution.

Approximately 450.72 mg of free crystal form A was weighed and placed into a glass vial, 378.605 mL of 0.5% CMC-Na aqueous solution was added, the mixture was stirred and sonicated until particles were dispersed, to obtain a white suspension at a final concentration of 1.2 mg/mL. The preparations were freshly prepared on the day of administration, and sampled for determination of preparation concentration.

### 4. Experimental groups

Six Beagle dogs (half male and half female) were fasted overnight in metabolic cages the day before the experiment. Before administration, urine and feces of each animal were collected as baseline (0 h) samples. On the day of administration, Beagle dogs received single intragastric administration of the drug at 6 mg/kg. Four hours after administration, the animals resumed feeding and had free access to water during the experiment.

### 5. Sample collection and treatment

Urine and feces excretion experimental group: urine and feces were collected before administration and 0 to 4 h, 4 h to 8 h, 8 h to 12 h, 12 h to 24 h, 24 h to 48 h, 48 h to 72 h, 72 h to 96 h, and 96 h to 120 h after administration. After urine collection is completed, the volume was measured and recorded. Part of the collected urine was taken, methanol was added to dilute the urine in a urine-to-methanol volume ratio of 4: 1 (v/v). After vortex mixing, the mixture was divided into 2 portions. After fecal samples at different time periods were collected, the food residues on the surface were removed and the feces were weighed. A certain volume of homogenate (20% methanol in water) was added according to the weight of feces in a feces-to-homogenate ratio of 1: 10 (feces weight: homogenate volume, w/v), and the mixture was homogenized, and divide into 2 portions. After the last sampling of the urine and feces excretion experimental group was completed, the metabolic cages were rinsed with 2000 mL of water: ethanol (1: 1, v: v) and the rinse solution of each cage was collected. After the rinse solution was thoroughly shaken, 1 mL was immediately taken, placed into a 1.5 mL EP tube, and stored in a -90°C to 60°C refrigerator together with the urine and feces homogenate until sample analysis.

The drug concentration in the excretion samples of the animal in each group at different time periods was detected by LC-MS/MS, and an excretion rate and a cumulative excretion rate in each time period were calculated.

### 6. Experimental results

**Table 32 Cumulative excretion rate of Beagle dog receiving single intragastric administration of drug at 6 mg/kg**

| **Cumulative excretion rate (%)** | | | |
|---|---|---|---|
| Excretion sample | Urine (0 to 120 h) | Feces (0 to 120 h) | Rinse solution (120 h) |
| Mean value | 43.8 | 3.48 | 4.87 |
| SD | 9.97 | 0.663 | 3.09 |

After the Beagle dog receives single intragastric administration of the drug at 6 mg/kg, the cumulative excretion rate of the drug in the urine of the Beagle dog within 120 h is 43.8±9.97%, and the cumulative excretion rate in the feces within 120 h is 3.48±0.663%. The total cumulative excretion rate of the drug in feces and urine of the Beagle dog within 120 h is 47.3±9.62%. After 120 h, the amount of drug in the cage rinse solution of the Beagle dog is 4.87±3.09% of the administered dose. After the experimental dog received the drug Apoquel^{®} orally, a cumulative excretion rate of Apoquel^{®} in dog urine within 24 h is only 3.6% (P8, CVMP assessment report for APOQUEL (EMEA/V/C/002688/0000), EMA/481054/2013).

In conclusion, after single intragastric administration of the drug at 6 mg/kg, the total excretion rate of the drug of the present application in the Beagle dog is 52.2% (the feces, the urine, and the rinse solution), indicating that the free crystal form A is more stable in dogs.

Finally, it should be noted that the foregoing embodiments are only used to illustrate the technical solutions of the present application, rather than to limit it. Although the present application has been described in detail with reference to the foregoing embodiments, those skilled in the art should understand that they may still modify the technical solutions described in the foregoing embodiments, or replace some or all of the technical features with equivalents. However, these modifications or replacements do not make the essence of the corresponding technical solutions deviate from the scope of the technical solutions in the embodiments of the present application.

## Claims

1. A crystal form I of a compound represented by formula I, wherein an X-ray powder diffraction (XRPD) pattern of the crystal form I exhibits characteristic peaks at positions corresponding to at least three 2θ values selected from approximately 12.4°±0.2°, 14.6°±0.2°, 16.7°±0.2°, 17.2°±0.2°, 20.3°±0.2°, 24.8°±0.2°, and 25.0°±0.2°

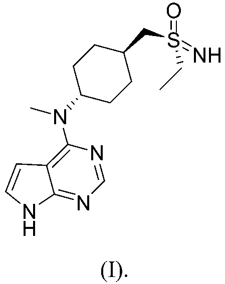

2. The crystal form I according to claim 1, wherein the XRPD pattern of the crystal form I further exhibits characteristic peaks at positions corresponding to at least three 20 values selected from approximately 13.2°±0.2°, 15.2°±0.2°, 19.3°±0.2°, 21.2°±0.2°, 21.6°±0.2°, 24.0°±0.2°, and 27.0°±0.2°.

3. The crystal form I according to claim 1, wherein the crystal form I has an XRPD pattern substantially same as that shown in FIG. 1; and preferably, the crystal form I has a differential scanning calorimetry (DSC) pattern and a thermal gravimetric analysis (TGA) pattern that are substantially the same as those shown in FIG. 2.

4. A preparation method of the crystal form I according to any one of claims 1 to 3, comprising: taking an amorphous compound represented by formula I as a starting material to prepare the crystal form I, the method being selected from any one or a combination of an antisolvent addition method, a gas-solid diffusion method, a suspension stirring method, a slow volatilization method, a slow cooling method, a gas-liquid permeation method, and anti-antisolvent addition method;
preferably, the antisolvent addition method comprising: dissolving an amorphous raw material of the compound represented by formula I in a good solvent, and adding an antisolvent;
preferably, the good solvent being selected from one of methanol, 1,4-dioxane, trichloromethane, and dimethyl sulfoxide;
preferably, the antisolvent being selected from one of methyl isobutyl ketone, isopropyl acetate, methyl tert-butyl ether, n-heptane, ethyl acetate, meta-xylene, cyclopentyl methyl ether, toluene, anisole, and water; and
more preferably, the good solvent being methanol, and the antisolvent being selected from methyl isobutyl ketone, isopropyl acetate, and methyl tert-butyl ether; or the good solvent being 1,4-dioxane, and the antisolvent being selected from n-heptane and ethyl acetate; or the good solvent being trichloromethane, and the antisolvent being selected from n-heptane, meta-xylene, and cyclopentyl methyl ether; or the good solvent being dimethyl sulfoxide, and the antisolvent being selected from toluene, anisole, and water.

5. A hydrochloride crystal form I of a compound represented by formula I, wherein an XRPD pattern of the hydrochloride crystal form I exhibits characteristic peaks at positions corresponding to at least three 20 values selected from approximately 6.2°±0.2°, 10.9°±0.2°, 12.3°±0.2°, 16.3°±0.2°, 17.2°±0.2°, 18.9°±0.2°, 19.4°±0.2°, 24.7°±0.2°, and 27.5°±0.2°

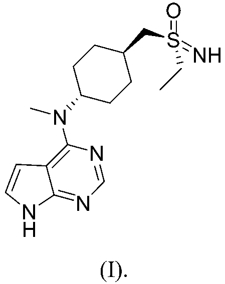

6. The hydrochloride crystal form I according to claim 5, wherein the XRPD pattern of the hydrochloride crystal form I further exhibits characteristic peaks at positions corresponding to at least three 20 values selected from approximately 11.6°±0.2°, 15.0°±0.2°, 18.4°±0.2°, 21.3°±0.2°, 23.3°±0.2°, 24.3°±0.2°, 25.6°±0.2°, 26.8°±0.2°, and 30.0°±0.2°.

7. The hydrochloride crystal form I according to claim 5, wherein the hydrochloride crystal form I has an XRPD pattern substantially same as that shown in FIG. 4.

8. The hydrochloride crystal form I according to claim 5, wherein the hydrochloride crystal form I has a DSC pattern and a TGA pattern that are substantially the same as those shown in FIG. 5.

9. A preparation method of the hydrochloride crystal form I according to any one of claims 5 to 8, comprising: mixing the crystal form I according to claim 1 with hydrochloric acid, and adding a solvent to obtain the hydrochloride crystal form I,
preferably, the solvent being selected from one of ethanol, acetone/water, ethyl acetate, and 2-methyltetrahydrofuran.

10. A pharmaceutical composition, comprising the crystal form I according to any one of claims 1 to 3 or the hydrochloride crystal form I according to any one of claims 5 to 8, and one or more pharmaceutically acceptable excipients.

11. Use of the crystal form I according to any one of claims 1 to 3 or the hydrochloride crystal form I according to any one of claims 5 to 8 in preparation of a drug for treating a disease associated with Janus kinase-signal transducer and activator of transcription (JAK-STAT) pathway.

12. Use of the crystal form I according to any one of claims 1 to 3 or the hydrochloride crystal form I according to any one of claims 5 to 8 in preparation of drugs for preventing and/or treating an inflammatory disease, a tumor, an autoimmune disease, and an allergic disease in humans and/or animals,
preferably, the inflammatory disease being selected from rheumatoid arthritis, canine dermatitis, psoriasis, ulcerative colitis, and Crohn's disease;
preferably, the tumor being selected from myelofibrosis, polycythemia vera, essential thrombocythemia, chronic myelogenous leukemia, breast cancer, lung cancer, and pancreatic cancer;
preferably, the autoimmune disease being selected from systemic lupus erythematosus, type 1 diabetes, rheumatoid arthritis, multiple sclerosis, ankylosing spondylitis, psoriasis, coeliac disease, ulcerative colitis, and Crohn's disease;
preferably, the allergic disease being selected from allergic dermatitis, allergic conjunctivitis, allergic asthma, and allergic rhinitis; and
more preferably, the allergic disease being canine and feline allergic dermatitis, which having one or more of the following symptoms: cutaneous pruritus, rash, hair loss, desquamation, edema, ulcer, especially canine allergic cutaneous pruritus.
